(19) Europäisches Patentamt
European Patent Office
Office européen des brevets

(11) Publication number: 0 007 204 B1

# (12) EUROPEAN PATENT SPECIFICATION

(45) Date of publication of patent specification: 24.11.82

(21) Application number: 79301279.0

(22) Date of filing: 02.07.79

(51) Int. Cl.³: C 07 C 91/30, A 23 K 1/16, A 61 K 31/135, A 61 K 31/165, A 61 K 31/215, C 07 C 93/26, C 07 C 101/42, C 07 C 103/28, C 07 C 103/76

## (54) Phenethanolamines, their formulations, use and preparation.

(30) Priority: 03.07.78 US 921670

(43) Date of publication of application: 23.01.80 Bulletin 80/2

(45) Publication of the grant of the patent: 24.11.82 Bulletin 82/47

(84) Designated Contracting States: DE GB IT NL SE

(56) References cited:
DE - A - 1 643 224
US - A - 4 000 192

(73) Proprietor: ELI LILLY AND COMPANY
307, East McCarty Street
Indianapolis Indiana 46285 (US)

(72) Inventor: Mills, Jack
7902, Timberhill Drive
Indianapolis, Indiana 46217 (US)
Inventor: Schmiegel, Klaus Kurt
4507 Staughton Drive
Indianapolis, Indiana 46226 (US)
Inventor: Shaw, Walter Norman
4511 North Broadway
Indianapolis, Indiana 46205 (US)

(74) Representative: Crowther, Terence Roger
European Patent Attorney et al,
Erl Wood Manor
Windlesham Surrey GU20 6PH (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Phenethanolamines, their formulations, use and preparation

This invention relates to phenethanolamine derivatives, pharmaceutical formulations containing those compounds, methods of preparing the novel compounds and their use in weight control in mammals.

In recent years very extensive research efforts have been effected in the area of β-phenylethylamine derivatives, such as catecholamines. A great deal of study, for example, has been carried out on the naturally occurring catecholamine epinephrine. Epinephrine is a potent sympathomimetic drug and a powerful cardiac stimulant. However, the use of epinephrine is limited because of its undesirable side effects, which include fear, anxiety, tremor, tenseness, throbbing headache, increased blood pressure, dizziness, respiratory difficulty and palpitation, as well as its short duration of action.

Use of drugs which cause more than one biological effect is always potentially dangerous. For example, since both bronchodilation and cardiac stimulation are mediated by the broad group of receptors known as β-receptors, a drug acting on such β-receptors not only would effect bronchodilation, but additionally could cause observable effects upon the heart. In fact, allegedly some individuals have died from ventricular fibrillation caused by excessive β-stimulation after using bronchodilation agents; (See Greenburg and Pines, *Br. Med. J. 1,* 563 (1967).

The cardiac activity of phenethanolamines has long been known and is well-documented, see for instance U.S. Patent Specification No. 3,816,516.

It has now surprisingly been discovered that the R,S-stereoisomer of certain N-substituted 3-phenylpropylamine derivatives are effective in weight control in obese mammals by removing, or preventing the formation of, excess adipose tissue, and further that such compounds possess minimal cardiac activity.

According to the present invention there is provided a compound of the formula (I):

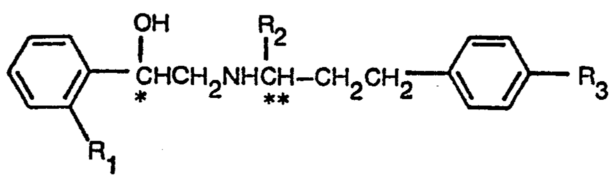

(I)

wherein:

$R_1$ is hydrogen of fluorine;

$R_2$ is methyl or ethyl;

$R_3$ is hydroxy, $C_1$—$C_4$ alkanoyloxy, aminocarbonyl, methylaminocarbonyl or $C_1$—$C_2$ alkoxycarbonyl;

$\underset{*}{C}$ is an asymmetric carbon atom having the R absolute stereochemical configuration; and

$\underset{**}{C}$ is an asymmetric carbon atom having the S absolute stereochemical configuration;

or a pharmaceutically-acceptable acid addition salt thereof.

A preferred group of compounds have the above formula wherein $R_2$ is methyl.

Additionally preferred compounds have the above formula wherein $R_1$ is hydrogen, $R_2$ is methyl and $R_3$ is hydroxy, methoxycarbonyl, aminocarbonyl or methylaminocarbonyl.

The most preferred compounds of formula (I) are those wherein $R_1$ is hydrogen, $R_2$ is methyl and $R_3$ is hydroxy or aminocarbonyl, and the pharmaceutically acceptable acid addition salts thereof.

In one aspect of the invention there is provided a pharmaceutical formulation which comprises a compound of formula (I), or a pharmaceutically-acceptable acid addition salt thereof, associated with a pharmaceutically-acceptable carrier therefor. A preferred formulation comprises a compound of the above formula wherein $R_2$ is methyl. An especially preferred pharmaceutical formulation comprises an R,S phenethanolamine of the above formula wherein $R_1$ is hydrogen, $R_2$ is methyl and $R_3$ is hydroxy or aminocarbonyl.

The invention also provides a compound of formula (I), as defined above, or a pharmaceutical formulation thereof, for use in removing, or preventing the formation of adipose tissue in animals.

Whilst the compounds provided by this invention may be referred to generically as phenethanolamines, they will be systematically named herein as N-substituted-3-phenylpropylamines. The stereochemical configuration of the compounds of this invention is designated according to the R and S nomenclature. A full discussion of this system of nomenclature is presented by Cahn *et al., Experientia,* Vol. XII, pp. 81—124 (1956). The stereochemical configuration of the carbon atom labeled "$\underset{*}{C}$" is R and will be presented first when naming the compounds of the invention. The asymmetric

carbon atom labeled "$\underset{**}{\text{C}}$" has the S absolute stereochemical configuration. Such fact is designated in the systematic naming of such compounds as illustrated for the compound of the above formula wherein $R_1$ is hydrogen, $R_2$ is methyl and $R_3$ is aminocarbonyl: R,S-N-(2-phenyl-2-hydroxyethyl)-1-methyl-3-(4-aminocarbonylphenyl)propylamine.

The compounds provided by this invention can be prepared by any of a number of methods involving well known and routinely used chemical processes. A preferred process involves the reaction of an optically active styrene oxide with an optically active 1-alkyl-3-phenylpropylamine.

According to a further aspect of the invention there is provided a method of preparing a compound of formula (I) in which a styrene oxide of formula (II):

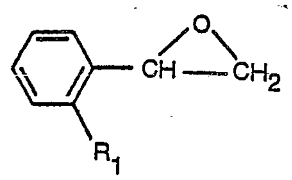

(II)

where $R_1$ is as defined above, is reacted with a propylamine derivative of formula (IV):

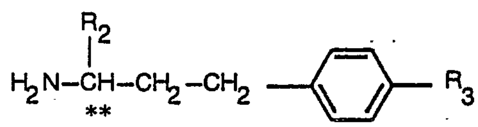

(IV)

where $R_2$ and $R_3$ are as defined above, followed in the case where the styrene oxide is not optically active, i.e. is not the R-styrene oxide, by resolution.

The optically active amines of formula (IV) can be prepared from the corresponding ketones of formula:

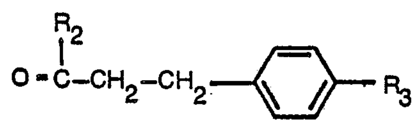

by reaction with (—)-$\alpha$-methylbenzylamine in the presence of an acid such as *p*-toluenesulfonic acid to provide the corresponding S—N-($\alpha$-methylbenzyl)-1-($R_2$)-3-(4-($R_3$)phenyll)-propylimine. Reduction of this imine with Raney nickel gives

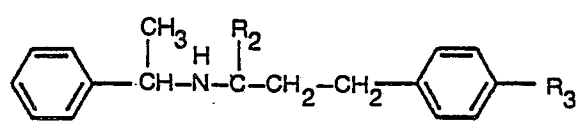

The above amine is salified, for example with HCl, and the salt purified by repeated crystallization from a suitable organic solvent. The optically active S,S—N-($\alpha$-methylbenzyl)-1-($R_2$)-3-(4-($R_3$)-phenyl)-propylaminium salt thus obtained can then be hydrogenated with palladium on charcoal to remove the $\alpha$-methylbenzyl group and liberate the amine of formula IV in salt form.

An optically active styrene oxide such as R-*ortho*-fluorostyrene oxide can be reacted with approximately an equimolar quantity of an optically active phenylpropylamine such as S-1-methyl-3-(4-methylaminocarbonylphenyl)propylamine to provide the corresponding optically active phenethanolamine of this invention, namely R,S—N-[2-(2-fluorophenyl)-2-hydroxyethyl]-1-methyl-3-(4-methylaminocarbonylphenyl)propylamine. Condensation reactions of this type can be carried out in an unreactive polar organic solvent such as ethanol, dioxane, toluene or dimethylformamide, and usually at a temperature of from about 20 to about 120°C, preferably from 50 to 110°C. Under such conditions, the condensation generally is substantially complete after about 6 to about 10 hours, and the product phenethanolamine can be isolated by simply removing the reaction solvent, for instance by evaporation under reduced pressure. Further purification of the product thus formed can be accomplished if desired by standard methods including chromatography, crystallization and salt formation.

Another preferred method for preparing the compounds of this invention comprises reacting an optically active mandelic acid derivative with a phenylpropylamine to provide an amide, and then reducing the amide carbonyl group to provide a compound of the invention. Such a method is ideally

suited to the preparation of compounds wherein $R_3$ is a hydroxy or alkoxycarbonyl substituent, but is not preferred for compounds in which $R_3$ is an amide group since a mixture of products may result.

According to further aspect of the invention there is provided a method of preparing a compound of formula (I) which comprises reducing a compound of formula (III)

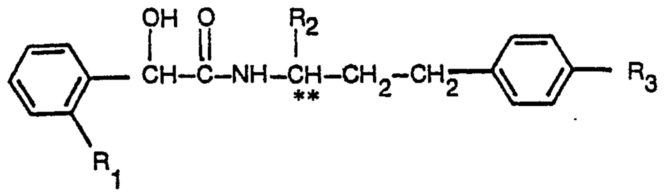

(III)

where $R_1$ and $R_2$ are as defined above and where $R_3$ is also as defined above or benzyloxy, followed in the case where $R_3$ is benzyloxy by hydrogenolysis of the benzyl group to give the compound of formula (I) where $R_3$ is hydroxyl, and, where the carbon atom at —CHOH— is unresolved by resolution to the R-enantiomer.

The reaction of the mandelic acid derivative with the phenylpropylamine is best accomplished utilizing coupling reagents such as those commonly used in the synthesis of peptides. Such routinely used coupling reagents include carbodiimides such as N,N′-dicyclohexylcarbodiimide (DCC) and N,N′-diisopropylcarbodiimide, as well as the N-ethoxycarbonyl-2-ethoxy-1,2-dihydroquinone (EEDQ). Dicyclohexylcarbodiimide generally is a preferred coupling reagent. A substituted or unsubstituted 1-hydroxybenzotriazole can be employed to accelerate the reaction if desired. The general process for preparing peptides utilizing dicyclohexylcarbodiimide and 1-hydroxybenzotriazole is discussed in detail by Konig and Geiger in *Chem. Ber., 103,* 788—798 (1970). According to such preferred process for preparing a compound of this invention, either R-2-phenyl-2-hydroxyacetic acid or R-2-(2-fluorophenyl)-2-hydroxyacetic acid can be reacted with approximately an equimolar quantity of an optically active S-1-alkyl-3-phenylpropylamine in the presence of a substantially equimolar amount of both dicyclohexylcarbodiimide and 1-hydroxybenzotriazole. The coupling reaction is best carried out in an organic solvent such as dimethylformamide, hexamethylphosphorotriamide, acetonitrile, dichloromethane, and the like. The reaction is normally carried out at reduced temperatures, for example at a temperature below about 50°C., generally at about 0 to about 10°C. The reaction normally is substantially complete within about two to about twenty hours; however, longer reaction periods appear not to be detrimental to the desired product and can be utilized if necessary.

The coupling of an acid and an amine in the presence of dicyclohexylcarbodiimide to form an amide converts the dicyclohexylcarbodiimide to dicyclohexylurea. This latter compound is characteristically quite insoluble in organic solvents and accordingly can be removed from the reaction mixture simply by filtration. Once the dicyclohexylurea is removed from the reaction mixture, the product amide can be isolated by removal of the reaction solvent, for instance by evaporation of the solvent under a reduced pressure. The product thus formed can be dissolved in a water immiscible organic solvent such as ethyl acetate and washed with aqueous sodium bicarbonate to remove any remaining 1-hydroxybenzotriazole. The amide so formed can, if desired, be further purified by routine procedures including crystallization and chromatography.

The amide next is reduced to provide the corresponding optically active N-phenylpropyl phenethanolamine comprehended by this invention. Reduction of such amide can be accomplished by any of a number of routine reduction processes, such as reaction with metal hydride reducing agents. A preferred reduction process comprises reaction of an amide with diborane. For example, an amide such as R,S—N-[2-(2-fluorophenyl)-2-hydroxy-1-oxoethyl]-1-ethyl-3-(4-hydroxyphenyl)propylamine can be reduced with diborane to provide a compound of formula (I). In this reduction the diborane typically is utilized in an excess relative to the amide, such as from about 1 to about 4 molar excess. The reduction generally is carried out in an inert organic solvent such as tetrahydrofuran, diethyl ether, benzene, dichloromethane, toluene or dioxane. Such reductions typically are complete within about two to twenty hours when carried out at a temperature of from about 0° to about 100°C. Any excess diborane and borane complex remaining in the reaction mixture after the reduction of the amide is complete can be decomposed by the addition to the reaction mixture of an alcohol such as methanol or ethanol and an acid such as hydrochloric acid. The reduced product thus formed can be isolated by simply removing the reaction solvent, for example by evaporation. The product, an optically active phenethanolamine having the above formula, generally exists as a solid and can thus be further purified by crystallization or chromatography. Alternatively, the amine so formed can be converted to an acid addition salt, as will be discussed more fully hereinbelow.

It should be recognized that the above-described processes for preparing compounds of formula (I) can be carried out utilizing starting materials unresolved at $\underset{*}{C}$, thereby affording mixtures of diastereomers of S,N-phenylpropyl phenethanolamines. Separation of the diastereomers so formed can be accomplished by normal processes when required. Whilst the R-optical isomer of the phenethanol portion of compounds having the above formula (i.e. $\underset{*}{C}$) is required for useful biological activity, it is not

a major disadvantage to have such R-isomer in admixture with the corresponding S-isomer (i.e. at $\underset{*}{C}$), since the S-isomer is substantially devoid of activity and causes no undesired side effects in biological systems at therapeutic doses. However, it is to be clearly understood that this invention comprehends only compounds of formula (I) wherein the asymmetric center labelled $\underset{**}{C}$ has the S absolute stereochemical configuration, since the R optical isomers (at the $\underset{**}{C}$ asymmetric center) are potent inotropic agents and cannot be used to treat obesity without simultaneously imparting a significant cardiac effect.

Use of the unresolved (at $\underset{*}{C}$) material may be particularly desirable if the compounds of the invention are to be utilized in the improvement of the quality of meat in livestock animals, such as cattle, pigs and sheep, by removing, or preventing the formation of, excess adipose tissue therein and the present invention provides an animal feedstuff comprising a compound of formula (I) as defined above, either alone or associated with the S,S-isomer. The compounds of the invention have particular value in improving the quality of pig carcasses, an animal which tends to fat. In such a method the mixture is preferably applied in an amount of from 5 to 250 mg./kg. per day—usually in the animal's feed.

Another method of preparing compounds of the invention comprises reacting a phenethanolamine with an aldehyde or ketone to provide the corresponding Schiff base, followed by reduction of the Schiff base. Such process is illustrated by the following scheme:

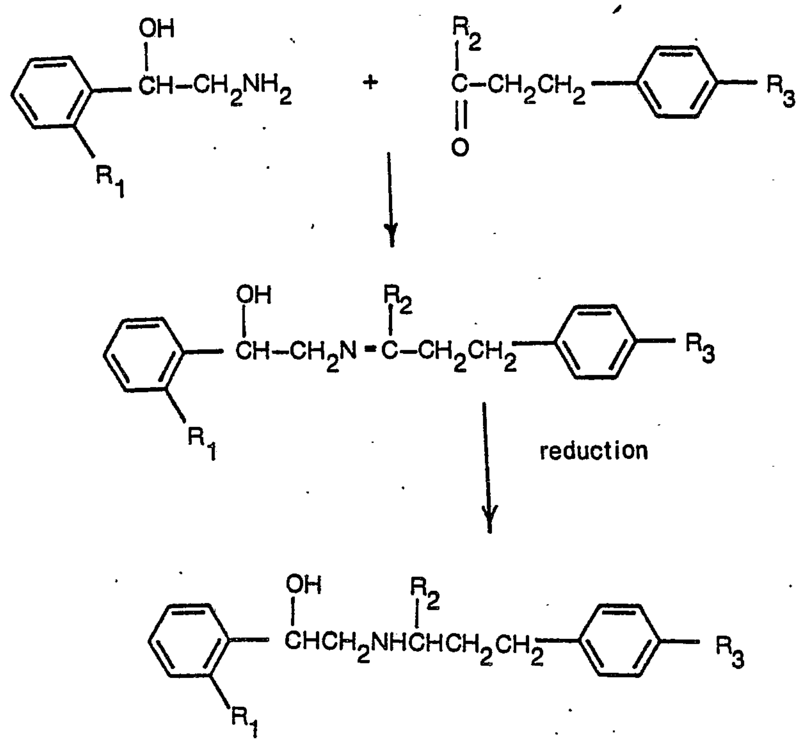


Generally, optically active R-phenethanolamines should be used in the reaction. For example, a compound such as R-2-phenyl-2-hydroxyethylamine can be condensed with an equimolar quantity of a ketone such as methyl 2-(4-ethoxycarbonylphenyl)ethyl ketone. Such condensation of a phenethanolamine and a phenethyl ketone can be carried out in an organic solvent such as toluene, generally in the presence of an acidic catalyst such as *para*-toluenesulfonic acid. The product from the condensation is an imine, i.e., a Schiff base, which upon reduction provides the corresponding phenethanolamines of the invention. Such reduction typically can be carried out with hydrogen or with a metal hydride such as sodium cyanoborohydride. The reduction of the imine affords a mixture of optical isomers at $\underset{**}{C}$, the separation of which can be accomplished utilizing standard resolution techniques such as fractional crystallization.

Still another method for preparing the compounds of this invention comprises reacting a hydroxy protected 2-phenyl-2-hydroxyacetic acid acylating agent with an optically active 1-alkyl-3-phenylpropylamine to provide an amide, followed by reduction of the amide carbonyl group to provide a compound of this invention. This process may be depicted by the following reaction scheme:

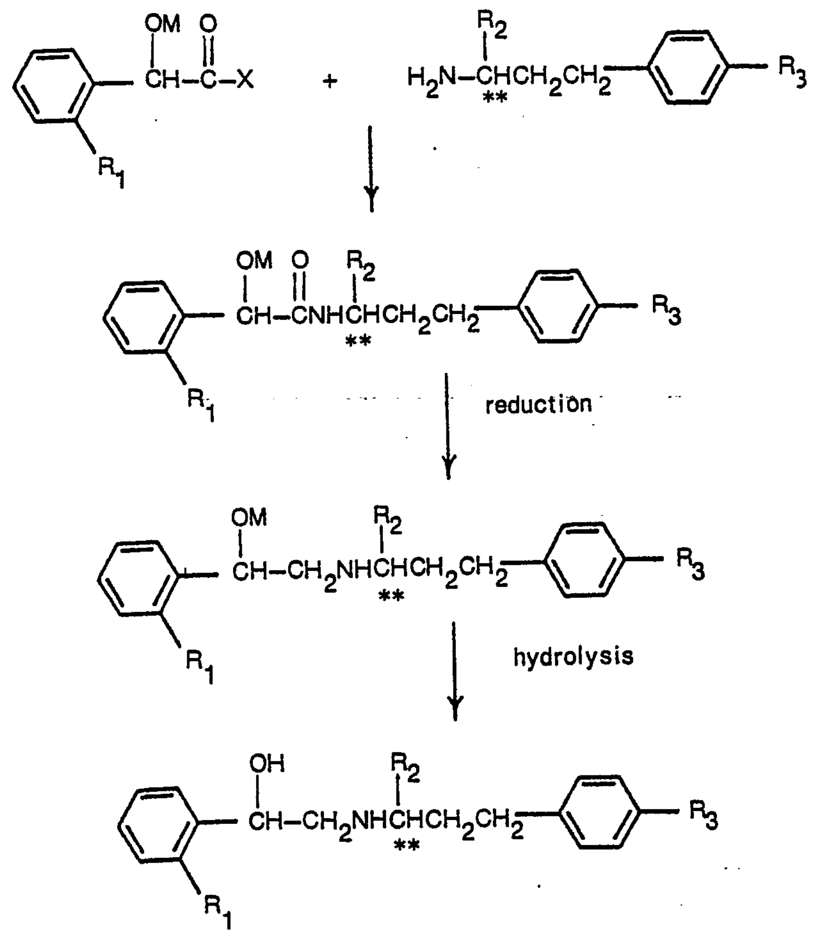


$R_1$, $R_2$ and $R_3$ in the above formulae are as defined above, X is as good leaving group and M is a readily removable hydroxy protecting group. Commonly used hydroxy protecting groups include acyl moieties such as acetyl, chloroacetyl and dichloroacetyl, as well as ether forming groups such as trimethylsilyl and the like. Such readily removable hydroxy protecting groups are more fully described by E. Haslam in *Protective Groups in Organic Chemistry,* J. F. W. McOmie, Ed., Plenum Press, New York, N.Y., 1973, Chapter 3. As noted above, X is defined as a good leaving group and includes halogen radicals such as chloride and bromide, as well as acyloxy groups such as acetoxy and dichloroacetoxy. As an example of the preparation of a compound of this invention utilizing a hydroxy protected mandelic acid acylating agent, a compound such as R-2-(2-fluorophenyl)-2-hydroxyacetic acid can be reacted with dichloroacetyl chloride to provide R-2-(2-fluorophenyl)-2-dichloroacetoxyacetic acid. Reaction of this latter compound with halogenating agent such as thionyl chloride or oxalyl chloride provides the corresponding acid chloride, namely R-2-(2-fluorophenyl)-2-dichloroacetoxyacetyl chloride. Reaction of this acid chloride with a phenylpropylamine, for instance S-1-methyl-3-(4-hydroxyphenyl)-propylamine, provides the corresponding amide, which amide in this case would be R,S—N-[2-(2-fluorophenyl)-2-dichloroacetoxy-1-oxoethyl]l-1-methyl-3-(4-hydroxyphenyl)propylamine. The amide so formed is next reduced, for example with diborane, and the hydroxy protecting group is removed, for instance by hydrolysis, thus providing an optically active compound of formula (I).

A further method of preparing compounds of formula (I) comprises reacting a phenacyl halide with an optically active S-1-alkyl-3-phenylpropylamine. For example, a phenacyl halide such as 2-fluorophenacyl bromide can be reacted with about an equimolar quantity of an amine such as S-1-methyl-3-(4-hydroxyphenyl)propylamine. Such alkylation reactions are normally carried out in an organic solvent such as ethanol and in the presence of a base such as sodium carbonate or triethylamine. The reaction is complete within about six hours when carried out at about 50°C. and provides a ketone intermediate, for instance S—N-[2-(2-fluorophenyl)-2-oxoethyl]-1-methyl-3-(4-hydroxyphenyl)propylamine. The ketone thus formed can be isolated as the free amine base or as an acid addition salt. Reduction of the ketone, for example with sodium borohydride, affords a compound of formula (I), generally as a mixture of optical isomers at $\underset{*}{C}$. Such a mixture of R, S and S,S, optical isomers can be separated by standard procedures, or if desired can be utilized directly since the S,S isomer is essentially devoid of biological activity.

Compounds having the above formula wherein $R_3$ is hydroxyl, that is compounds of this invention which bear a phenolic hydroxyl group, can be readily acylated to provide compounds having the above formula in which $R_3$ is $C_1$—$C_4$ alkanoyloxy. As used herein, the term "$C_1$—$C_4$ alkanoyloxy" includes formyloxy, acetoxy, propionoxy, butyroxy, and isobutyroxy. The acylation of a compound of this invention having a phenolic hydroxyl group typically is accomplished by reacting the hydroxyl compound with an acylating agent such as the appropriate acid anhydride or acid halide for example the acid chloride or acid bromide. Typical acylating agents commonly employed include acetic anhydride, propionyl chloride, isobutyryl bromide and formic-acetic anhydride.

It may be desirable to protect the hydroxyl and amino groups of the phenethanolamine portion of the compound of this invention prior to acylation of the free phenolic hydroxyl group, thereby precluding any undesired side reactions. For example, a compound of this invention such as R,S—N-[2-(2-fluorophenyl)-2-hydroxyethyl]-1-ethyl-3-(4-hydroxyphenyl)propylamine can be reacted with thionyl chloride in the presence of a base such as triethylamine and in a solvent such as tetrahydrofuran to form a cyclic oxathiazole, thus effectively protecting both the hydroxyl group and the amino group of the phenethanolamine portion of the molecule. Such reaction converts the above-named compound into R,S—N-[1-ethyl-3-(4-hydroxyphenyl)propyl]-5-(2-fluorophenyl)-1-oxo-4,5-dihydro-1,2,3-oxathiazole. This latter compound can then be acylated at the free phenolic hyroxyl functionality without risk of affecting other sites in the molecule. For instance, R,S—N-[1-ethyl-3-(4-hydroxyphenyl)propyl]-5-(2-fluorophenyl)-1-oxo-4,5-dihydro-1,2,3-oxathiazole can be reacted with an acylating agent such as acetic anhydride or acetyl chloride in a solvent such as benzene, xylene or toluene and in the presence of a base such as triethylamine, generally at a temperature of about 50° to 100°C., thus effecting acylation of the phenolic hydroxyl group to provide R,S—N-[1-ethyl-3-(4-acetoxyphenyl)propyl]-5-(2-fluorophenyl)-1-oxo-4,5-dihydro-1,2,3-oxathiazole. The oxathiazole thus prepared is treated with an acid such as dilute mineral acid to remove the protecting groups and thus recover the desired phenethanolamine. For example, the above-named oxathiazole can be treated with 1 N hydrochloric acid at a temperature of about 30°C. to provide R,S—N-[2-(2-fluorophenyl)-2-hydroxyethyl]-1-ethyl-3-(4-acetoxyphenyl)propylamine as the acid addition salt, which upon treatment with a base such as sodium carbonate affords a free amine of formula (I).

Compounds of the formula (I) wherein $R_3$ is aminocarbonyl can be prepared either directly by reacting the appropriate phenylpropylamine with a styrene oxide or alternatively can be derived from those compounds wherein $R_3$ is methoxycarbonyl or ethoxycarbonyl. For example, R-mandelic acid can be coupled with S-1-methyl-3-(4-methoxycarbonylphenyl)propylamine to provide, after reduction of the amide carbonyl, the corresponding amine of this invention, namely, R,S—N-(2-phenyl-2-hydroxyethyl)-1-methyl-3-(4-methoxycarbonylphenyl)propylamine. This amine can be reacted with hydrazine for about 2 to 40 hours at about 50 to 100°C. to convert the alkoxycarbonyl group to a hydrazino carbonyl group. The latter compound is converted by hydrogenation to the corresponding aminocarbonyl derivative, utilizing common hydrogenation catalysts such as Raney nickel.

Compounds of formula (I) wherein $R_3$ is methylaminocarbonyl are preferably prepared by reacting a styrene oxide with a 3-(4-methylaminocarbonylphenyl)propylamine. The required phenylpropylamine starting material can be prepared by simply reacting methylamine with the appropriate benzoyl chloride derivative. For example, S-1-ethyl-3-(4-chlorocarbonylphenyl)propylaminium chloride can be reacted with methylamine to provide S-1-ethyl-3-(4-methylaminocarbonylphenyl)propylamine. Reaction of the latter compound with a styrene oxide affords a compound of this invention.

The compounds provided by this invention are amines and as such are basic in nature. Consequently, they can readily be converted to acid addition salts by reaction with organic or inorganic acids. Accordingly, an additional embodiment of this invention comprises pharmaceutically acceptable acid addition salts of the N-phenylpropyl phenethanolamines of the above formula. The particular acids utilized to form salts of this invention are not critical, and such salts include those which are prepared by reaction of the amine of this invention with any of a number of common acids such as hydrochloric, hydrobromic, sulfuric, phosphoric, nitric, perchloric, formic, acetic, butyric, citric, maleic, succinic, oxalic, fumaric, lactic, methane-sulfonic, p-toluenesulfonic, and related acids. The pharmaceutically acceptable acid addition salts which are formed by reaction of an amine of this invention with an acid such as one of the above-named acids typically exist as highly crystalline solids, and thus lend themselves to ready purification by recrystallization from common solvents such as methanol, ethanol, ethyl acetate and the like. Additionally, such salts are easily formulated for convenient administration, particularly by the oral route, to subjects in need of treatment for obesity. When desired, such acid addition salts are readily converted to the corresponding free amine base by reaction with a suitable basic compound, for instance sodium or potassium hydroxide, sodium carbonate, triethylamine, sodium bicarbonate, and the like.

The following list of compounds is provided to illustrate the range of compounds comprehended by this invention. The representative listing is not intended to be inclusive of every compound claimed herein, but rather simply illustrates certain preferred compounds and the relative scope of the disclosure.

R,S—N-(2-phenyl-2-hydroxyethyl)-1-methyl-3-(4-hydroxyphenyl)propylamine;

R,S—N-(2-phenyl-2-hydroxyethyl)-1-methyl-3-(4-aminocarbonylphenyl)propylamine;

R,S—N-(2-phenyl-2-hydroxyethyl)-1-ethyl-3-(4-hydroxyphenyl)propylamine;
R,S—N-(2-phenyl-2-hydroxyethyl)-1-ethyl-3-(4-aminocarbonylphenyl)propylamine;
R,S—N-(2-phenyl-2-hydroxyethyl)-1-methyl-3-(4-methylaminocarbonylphenyl)propylamine;
R,S—N-(2-phenyl-2-hydroxyethyl)-1-methyl-3-(4-acetoxyphenyl)propylamine;
R,S—N-[2-(2-fluorophenyl)-2-hydroxyethyl]-1-methyl-3-(4-ethoxycarbonylphenyl)propylamine;
R,S—N-[2-(2-fluorophenyl)-2-hydroxyethyl]-1-ethyl-3-(4-methoxycarbonylphenyl)propylamine;
R,S—N-(2-phenyl-2-hydroxyethyl)-1-methyl-3-(4-isobutyroxyphenyl)propylamine;
R,S—N-(2-phenyl-2-hydroxyethyl)-1-ethyl-3-(4-methylaminocarbonylphenyl)propylamine;
R,S—N-(2-phenyl-2-hydroxyethyl)-1-methyl-3-(4-ethoxycarbonylphenyl)propylamine;
R,S—N-(2-phenyl-2-hydroxyethyl)-1-methyl-3-(4-hydroxyphenyl)propylaminium chloride;
R,S—N-(2-phenyl-2-hydroxyethyl)-1-methyl-3-(4-methylaminocarbonylphenyl)propylaminium bromide;
R,S—N-(2-phenyl-2-hydroxyethyl)-1-methyl-3-(4-hydroxyphenyl)propylaminium acetate;
R,S—N-(2-phenyl-2-hydroxyethyl)-1-methyl-3-(4-hydroxyethyl)propylaminium butyrate;
R,S—N-(2-phenyl-2-hydroxyethyl)-1-methyl-3-(4-aminocarbonylphenyl)propylaminium succinate;

Obesity is a very serious disease that currently is the subject of a great deal of concern, particularly since no truly effective treatments are known. A full discussion of nutritional diseases and obesity in general is presented by Albrink in *Textbook of Medicine,* 12th Ed., 1969, W. B. Saunders Company, Philadelphia, Pa., pp. 1164—1174 and by Salans in *Current Therapy,* 1977, W. B. Saunders Company, pp. 455—460. Compounds provided by this invention are particularly useful due to their ability to effect an actual reduction in weight when administered to a mature obese animal. Such weight reduction is accomplished without a concomitant reduction in daily food consumption. When the compounds of this invention are administered to immature obese animals, the degree of weight gain is significantly reduced compared to that observed in young obese animals not receiving a compound of the invention.

The anti-obesity activity of the compounds of this invention has been demonstrated in a number of biological tests utilizing mice, rats and dogs. One of the major actions of the claimed compounds on a biological system appears to be the mobilization of fatty acids from adipose tissue stores. In a test designed to demonstrate such mobilization, a compound of this invention was administered to a total of eight normal fed rats of the Charles River strain, each weighing about 180 to 200 grams. A blood sample from each animal was taken immediately preceding the administration of the test compound and served as the control for each of the eight animals. A compound of this invention was then administered to each animal subcutaneously at the dose of 10 mg./kg. of body weight. Blood samples were then taken from each animal at intervals of 30, 60, 90 and 120 minutes after dosing, and the serum free fatty acid levels were determined for each blood sample. Table I presents the results of such test carried out with two compounds of this invention. The results demonstrate that a dramatic increase in serum free fatty acid levels is caused by a compound of this invention.

TABLE I

| | Average % of serum free fatty acid rise after losing |
|---|---|
| R,S—N-(2-phenyl-2-hydroxy)-1-methyl-3-(4-hydroxyphenyl)propylamine | 425% |
| R,S—N-(2-phenyl-2-hydroxyethyl)-1-methyl-3-(4-aminocarbonylphenyl)propylamine | 350% |

In a test designed to demonstrate the actual weight reducing effects of the compounds of this invention, viable yellow genetically obese mice were selected as a model. All of the animals were 6.5 months old at the beginning of the study. The animals were fed Purina Laboratory Chow and water *ad libitum* throughout the study. Five obese animals were randomly selected to receive 10 mg./kg. of R,S—N-(2-phenyl-2-hydroxyethyl)-1-methyl-3-(4-hydroxyphenyl)propylamine subcutaneously twice daily, and five animals received placebo. The initial body weight of each animal was determined on day 1 of the study prior to the first injections. Table II presents the results of the study. The data is presented as the average body weight in grams of the control group and of the group receiving the test compound. Such average body weight in grams is presented in column I under each of the indicated days. Column II under each of the indicated days provides the average food consumption in grams for the control group and for the test group.

The results presented in Table II demonstrate that the compounds of this invention cause an actual reduction in body weight in mature obese animals without causing a decrease in food consumption.

TABLE II

| | Day I | | Day 10 | | Day 20 | | Day 30 | | Day 40 | | Day 50 | |
|---|---|---|---|---|---|---|---|---|---|---|---|---|
| | I | II | I | II | I | II | I | II | I | II | I | II |
| Control group receiving placebo | 50 | 3.2 | 50 | 4.8 | 51 | 5.1 | 50 | 5.0 | 50 | 4.0 | 51 | 4.0 |
| test group receiving drug | 48 | 3.0 | 43 | 4.8 | 40 | 5.0 | 37 | 5.0 | 36.5 | 4.4 | 37 | 4.5 |

A similar study was carried out utilizing genetically obese Zucker rats. The compounds of the invention effected a dramatic weight reduction without decreasing daily food consumption when administered to adult obese rats (6 months and older). When administered to immature rats ranging in age from 2 to 5 months, the compounds were effective in preventing the excessive gain in weight as was observed with the young obese rats which did not receive a compound of this invention. Table III presents the results of a study carried out by administering R,S—N-(2-phenyl-2-hydroxyethyl)-1-methyl-3-(4-hydroxyphenyl)propylamine at 10 mg./kg. twice daily to the test animals. All animals were permitted to eat all the food and drink all the water they wanted. The food consumption of the animals given a compound of the invention did not differ significantly from the control animals receiving placebo. Each of the body weights reported in the table is the average weights of *five* animals in each of the test classes.

TABLE III

| | Day 1 | Day 20 | Day 40 | Day 60 | Day 80 |
|---|---|---|---|---|---|
| | body weight in grams | body weight in grams | body weight in grams | body weight in grams | body weight in grams |
| Immature Zucker controls | 490 | 500 | 525 | 550 | 570 |
| Immature Zucker rats receiving drug | 475 | 480 | 482 | 478 | 485 |
| Mature Zucker controls | 629 | 631 | 630 | 625 | 629 |
| Mature Zucker rats receiving drug | 632 | 625 | 595 | 575 | 560 |

The anti-obesity drugs provided by this invention additionally have demonstrated their ability to effect weight reductions in obese dogs. Obese breeder beagles from the Pin Oak colony ranging in age from 4 to 9 years and weighing from 15.4 to 29.0 kg. were selected for the test. The dogs were fed *ad libitum* a Eucanuba diet containing sixteen percent fat for six months prior to the initiation of the study, thereby stabilizing the dogs' obesity. In one test, the animals received R,S—N-(2-phenyl-2-hydroxyethyl)-1-methyl-3-(4-hydroxyphenyl)propylaminium chloride at a subcutaneous dose of 3.2 mg./kg. twice each day. The effect of the drug on body weight was determined by measuring body weight in kilograms. No decrease in food consumption was noted. The results of five weeks treatment are summarized in Table IV.

TABLE IV

| | Weight in Kg. Day 1 | Weight in Kg. Day 35 | Weight loss Kg. | percent weight loss |
|---|---|---|---|---|
| Dog 1 | 17.2 | 15.8 | 1.4 | 8% |
| Dog 2 | 29.2 | 25.5 | 3.7 | 12.5% |
| Dog 3 | 18.7 | 16.5 | 2.2 | 12% |
| Dog 4 | 16.2 | 15.0 | 1.2 | 7.5% |

In a similar test utilizing mature obese beagle hounds, fed *ad libitum* on standard dog chow, R,S—N-(2-phenyl-2-hydroxyethyl)-1-methyl-3-(4-aminocarbonylphenyl)propylaminium chloride was administered orally at 15 mg./kg. twice daily for twenty-seven days. The effect of the drug on body weight was determined by measuring body weight in kilograms and girth sizes in inches. Two dogs were selected as control animals receiving food and water *ad libitum,* but no drug. Table V presents the results of the test. As the data demonstrates, the control dogs did not fluctuate a great deal in weight or girth size. In contrast, the obese dogs treated with a compound of this invention lost from 1.8 to 2.9 kilograms during a 27 day treatment period, and the girth of such obese dogs decreased by as much as 5.08 cm (2 inches) in the same period of time.

TABLE V

| | Day 1 | | Day 7 | | Day 14 | | Day 21 | | Day 27 | |
|---|---|---|---|---|---|---|---|---|---|---|
| | Weight | girth size | Weight | girth size | Weight | girth size | Weight | girth size | Weight | girth size |
| | Kg. | cm (ins) | Kg. | cm (ins) | Kg. | cm (ins) | Kg. | cm (ins) | Kg. | cm (ins) |
| Control animal | 16.7 | 60.96 (24) | 16.2 | 62.23 (24.5) | 16.9 | 62.23 (24.5) | 16.2 | 63.5 (25) | 16.4 | 63.5 (25) |
| Control animal | 16.3 | 63.5 (25) | 16.4 | 63.5 (25) | 16.5 | 60.96 (24) | 16.5 | 60.96 (24) | 16.5 | 63.5 (25) |
| Test animal | 20.4 | 69.85 (27.5) | 20.5 | 68.58 (27) | 19.5 | 68.58 (27) | 18.1 | 68.58 (27) | 17.5 | 64.77 (25.5) |
| Test animal | 17.7 | 66.04 (26) | 16.9 | 66.04 (26) | 16.7 | 64.77 (25.5) | 15.9 | 66.04 (26) | 15.9 | 63.5 (25) |
| Test animal | 20.7 | 66.04 (26 | 20.0 | 66.04 (26) | 19.7 | 64.77 (25.5) | 19.8 | 63.5 (25) | 18.6 | 63.5 (25) |

As already pointed out, the unique physiological effect of the compounds of this invention is derived from their potent anti-obesity activity coupled with their physiologically tolerable degree of cardiovascular activity. This uniquely specific activity is achieved by selecting the correct, i.e. R,S—, stereochemistry at both the asymmetric carbon atoms of the compound of formula (I).

The unique physiological activity of the R,S-isomers of this invention compared with the corresponding R,R-isomers (which are potent inotropic agents) has been demonstrated in dogs. The compounds to be evaluated were administered intravenously to dogs having implanted cardiovascular transducers for measurement of the derivative of the left ventricular pressure, which is the index of cardiac contractile force. A dose of compound sufficient to cause a twenty-five percent increase in contractile force was administered. The dose required to effect such increase in contractile force is reported in Table VI as the $ED_{25}$ in mcg./kg. The results demonstrate that very little of the inotropically active R,R-isomers is required to effect a twenty-five percent increase in contractile force, whereas a significantly larger dose of the corresponding R,S-isomer is required to cause the same effect.

TABLE VI

| Compound administered | Contractile Force $ED_{25}$ mcg./kg. |
|---|---|
| R,S—N-(2-phenyl-2-hydroxyethyl)-1-methyl-3-(4-hydroxy-phenyl)propylamine | 100.0 |
| R,R,—N-(2-phenyl-2-hydroxy-ethyl)l-1-methyl-3-(4-hydroxy-phenyl)propylamine | 2.5 |
| R,S—N-(2-phenyl-2-hydroxyethyl)-1-methyl-3-(4-amino-carbonylphenyl)propylamine | 100.0 |
| R,R—N-(2-phenyll-2-hydroxy-ethyl)-1-methyl-3-(4-amino-carbonylphenyl)propylamine | 3.0 |

Because of the surprisingly low inotropic activity of the compounds of this invention, they can be administered to an animal at doses large enough to effect release of free fatty acids from adipose stores without causing a substantial increase in the pumping force of the heart. Such unique biological spectrum of the compounds of this invention renders them particularly useful in the control of weight in obese animals. As used herein, "weight control in obese animals" refers to the ability of the compounds of this invention to effect an actual weight reduction when administered to a mature obese animal, whereas such compounds are effective in the prevention of excessive weight gain when administered to immature obese animals. The terms "mature" and "immature" are used herein to refer to the generally accepted definitions of age and growth patterns. "Obesity" is an art recognized term and is used as such herein.

While the anti-obesity effective dose of a compound of this invention employed in the control of obesity will vary depending on the particular drug employed and the severity of the condition being treated, the usual dosage of a compound of this invention will be from about 1.0 to about 25 mg. per kilogram of animal body weight. The compounds of the invention preferably will be administered orally at a dosage of from about 1 to about 5 mg./kg., generally given in individual doses from one to four times per day. When desired, the drug can be administered orally in the form of a tablet or capsule, or alternatively in sustained release form. A compound defined by the above general formula is administered to a mature obese animal to effect an actual reduction in weight without diminishing the daily food consumption. The drug will be administered daily, at increasing dosage levels if desired, until the desired weight reduction is effected. The compounds of the invention can be administered to an immature obese animal to effect a reduction in weight gain without a diminished daily food consumption. Once the immature obese animal reaches maturity, a reduction of weight will be effected until a substantially normal weight is achieved.

The compounds comprehended by this invention can be formulated by normal procedures for convenient administration by any of a number of routes. It is preferred that the compounds be formulated for oral administration. Pharmaceutical formulations which are useful in weight control in obese animals are provided in a further embodiment of this invention.

Such pharmaceutical compositions can contain, as active ingredient, one or more of the compounds provided by this invention, in combination if desired with any of the inactive isomers as hereinbefore indicated, in addition to any of a number of pharmaceutically acceptable carriers or diluents. Typical carriers and diluents commonly used include gelatin, starch, dextrose, sucrose, lactose, cellulose derivatives, stearates, polyvinylpyrrolidine, glycerine, ethyl lactate, sorbitol, mannitol, and the like. A suitable pharmaceutical composition can additionally include any of a number of common preserving agents, stabilizers, antioxidants, taste correctors, and the like. Examples of such additives include ascorbic acid, sorbic acid, various esters of p-hydroxybenzoic acid, and the like.

Typical pharmaceutical compositions useful in the treatment of obesity according to this invention will generally include from about 1 to about 50 percent by weight of a compound of this invention as active ingredient. The remainder of said pharmaceutical compositions will comprise suitable carriers and diluents, and any of the indicated inactive optical isomers as desired.

A pharmaceutical composition containing as active ingredient at least one of the compounds of this invention can be molded into tablets, encapsulated into empty gelatin capsules, or made into a solution or suspension. Such pharmaceutical compositions can be administered to an obese subject in need of treatment by any of a number of routes, including the oral and parenteral routes. A preferred formulation, for example, comprises about 250 mg. of R,S—N-(2-phenyl-2-hydroxyethyl)-1-methyl-3-(4-aminocarbonylphenyl)propylaminium admixed with any of a number of suitable carriers and molded into a tablet for oral administration to an obese human subject at the rate of from 1 to about 4 tablets per day for effective weight control.

In an effort to more fully illustrate particular aspects of this invention, the following Preparations illustrate how starting materials useful in the invention may be prepared whereas the following Examples illustrate the preparation of final products of formula (I). The Examples are representative only and should not be construed as limiting in any respect. In the Preparations and Examples, the optical rotations were measured at room temperature and at a concentration of 9.3 mg of compound per ml. of solvent.

### Preparation 1
### Preparation of R-styrene oxide

To a cold stirred solution of 300 g. of R-mandelic acid in 2000 ml. of tetrahydrofuran was added dropwise over two hours 3000 ml. of a 1 molar solution of diborane in tetrahydrofuran. The reaction mixture was stirred for twelve hours at 25°C. following complete addition, and then was cooled to 0°C. in an ice water bath. To the cold solution was added 600 ml. of methanol dropwise over thirty minutes, and the reaction mixture was then stirred for another three hours. Removal of the solvent by evaporation under reduced pressure provided 260 g. of the product as a solid. The solid was crystallized from diethyl ether to provide R-2-phenyl-2-hydroxyethanol.

A solution of 256.7 g. of R-2-phenyl-2-hydroxyethanol in 1000 ml. of toluene containing 50 ml. of pyridine was stirred at 0°C. while a solution of 372.1 g. of *p*-toluenesulfonyl chloride in 400 ml. of toluene was added dropwise over two hours. The reaction mixture was stirred at 0°C. for forty-eight hours and then filtered to remove the precipitated pyridine hydrochloride. The filtrate was concentrated to dryness by evaporation under reduced pressure to provide the product as an oil. The oil was dissolved in fresh toluene, washed with dilute hydrochloric acid and with water, and dried. Evaporation of the solvent under reduced pressure and crystallization of the product from diethyl ether and hexane afforded 435 g. of R-2-phenyl-2-hydroxy-1-*p*-toluenesulfonyloxyethane. The product thus formed was dissolved in 1000 ml. of dimethyl sulfoxide containing 418 ml. of 5 N sodium hydroxide. The alkaline solution was stirred at 0°C. for twelve hours, and then was poured into ice water. The product was extracted into 50% diethyl ether-pentane, and the organic layer was separated, washed with water and dried. Removal of the solvent by evaporation afforded, after distillation, 165 g. of R-styrene oxide. B.P. 55—56°C. 79.99 N/m² (0.6 torr). $[\alpha]_D$ −23.7° (chloroform).

### Preparation 2
### Preparation of R-*ortho*-fluorostyrene oxide

One hundred three grams of dl-*o*-fluoromandelic acid was resolved by reaction with (+)-$\alpha$-methylbenzylamine to form the salt, and crystallization of the salt from ethanol and ethyl acetate to provide optically active $\alpha$-methylbenzylaminium 2-(2-fluorophenyl-2-hydroxy)acetate, M.P. 155—157°C. $[\alpha]_D$ −43.1° (MeOH). The salt thus formed was hydrolyzed to the free acid to provide R-*ortho*-fluoromandelic acid, M.P. 88—90°C. $[\alpha]_D$ −138° (MeOH). The acid was then converted to R-*ortho*-fluorostyrene oxide by the procedure of Preparation 1, B.P. 58—61°C. 399.9 N/m² (3 torr), $[\alpha]_D$ −32.7° (chloroform).

### Preparation 3
### Preparation of S-1-methyl-3-(4-aminocarbonylphenyl)propylamine

The oil from 280 g. of a 50% dispersion of sodium hydride in mineral oil was removed by several washings with hexane. The hexane was replaced with 1.5 l. of dimethylformamide, and the mixture was stirred and cooled in an ice-bath while 950 g. of ethyl acetoacetate was slowly added. After the sodium

hydride had reacted a solution of 314 g. of α-bromo-p-toluic acid in 500 ml. of dimethylformamide was slowly added. The ice bath was removed and the mixture was allowed to stir at 25°C. for eighteen hours, poured into water acidified with 3N hydrochloric acid and extracted with ethyl acetate. The combined organic layers were washed with water and brine and dried over anhydrous sodium sulfate. The solvent and excess ethyl acetoacetate were removed at reduced pressure to afford 500 g. of oil.

The crude oil was combined with 1.2 l. of glacial acetic acid, 140 ml. of concentrated hydrochloric acid and 750 ml. of water and heated at refluxed for three and one half hours. The solvents were removed under reduced pressure and the residue was dissolved in a mixture of ether and xylene and washed with water. The solid obtained after removal of the solvents was recrystallized twice from a mixture of ethanol and water to give 111 g. of 4-(4-carboxyphenyl)-2-butanone, m.p. 120.5—123°C.

Analysis calc. for $C_{11}H_{12}O_3$

Theory: C, 68.74; H, 6.29.

Found: C, 68.99; H, 6.10.

A mixture of 9.50 g. of 4-(4-carboxyphenyl)-2 butanone, 100 ml. of benzene, 15.7 g. of oxalyl chloride and one drop of dimethylformamide was stirred and warmed to 50°C. for two hours. The reaction mixture was then allowed to stir for eighteen hours at 25°C. and concentrated under reduced pressure. The residue was dissolved in 50 ml. of dioxane and, with stirring, was added slowly to 300 ml. of cold (0°C.) concentrated ammonium hydroxide. The mixture was stirred for seventy-five minutes and diluted to 1 l. with water. The solid was removed by filtration, washed with water, dried and recrystallized from a mixture of methanol and ether to provide 4.1 g. of 4-(4-aminocarbonylphenyl)-2-butanone, m.p. 144—146°C. An analytical sample, m.p. 149—150°C., was obained by recrystallizing twice from a mixture of methanol and ether.

Analysis calc. for $C_{11}H_{13}NO_2$

Theory: C, 69.09; H, 6.85; N, 7.32.

Found: C, 68.98; H, 7.10; N, 6.99.

A solution of 45.7 g. of 4-(4-aminocarbonylphenyl)-2-butanone in 400 ml. of toluene containing 29.0 g. of (—)-α-methylbenzylamine and 1.0 g. of *p*-toluenesulfonic acid was heated at reflux for four hours with removal of water via a Dean Stark trap. After cooling the reaction mixture to room temperature, the solvent was removed therefrom by evaporation under reduced pressure to provide S—N-(α-methylbenzyl)-1-methyl-3-(4-aminocarbonylphenyl)-propylimine. The imine was hydrogenated in 625 ml. of ethanol utilizing 15 g. of Raney Nickel as the catalyst to provide a mixture of optical isomers of N-(α-methylbenzyl)-1-methyl-3-(4-aminocarbonylphenyl)propylamine. The amine was reacted with hydrogen chloride to provide the corresponding acid addition salt as a solid. The mixture was purified by repeated crystallization of the salt from methanol and acetonitrile to provide 23.6 g. of optically active S,S—N-(α-methylbenzyl)-1-methyl-3-(4-aminocarbonylphenyl)propylaminium chloride. M.P. 256—258°C. $[\alpha]_D$ —80.1° (MeOH).

A solution of 16.3 g. of the salt in 480 ml. of ethanol containing 3.0 g. of five percent palladium suspended on carbon was hydrogenated for twenty-two hours at 50°C. under a pressure of 60 p.s.i. The reaction mixture then was filtered and the solvent was removed from the filtrate by evaporation under a reduced pressure to provide the product as a solid. The solid was recrystallized three times from ethanol and ethyl acetate to afford 9.4 g. of S-1-methyl-3-(4-aminocarbonylphenyl)propylaminium chloride. M.P. 237—240°C. $[\alpha]_D$ —8.2 (MeOH).

Analysis calc. for $C_{11}H_{17}ClN_2O$

Theory: C, 57.76; H, 7.49; N, 12.25

Found: C, 57.65; H, 7.57; N, 12.10

Preparation 4

Preparation of S-1-methyl-3-(4-methylaminocarbonylphenyl)propylamine

Optically active S,S—N-(α-methylbenzyl)-1-methyl-3-(4-methoxycarbonylphenyl)propylamine was hydrolyzed by reaction with 10 percent aqueous hydrochloric acid to provide optically active S,S—N-(α-methylbenzyl)-1-methyl-3-(4-hydroxycarbonylphenyl)propylaminium chloride. M.P. 234—237°C. The acid thus formed was reacted with oxalyl chloride in benzene to provide the corresponding optically active acid chloride. The acid chloride was dissolved in dioxane and added dropwise to a stirred solution of methylamine in 50 ml. of dioxane. The reaction mixture was stirred for twelve hours at room temperature, and then diluted with 100 ml. of water. The aqueous reaction mixture was extracted with ethyl acetate, and the organic layer was separated, washed with water and dried. The solvent was removed by evaporation under reduced pressure to provide, after chromatography over silica gel, optically active S,S—N-(α-methylbenzyl)-1-methyl-3-(4-methylaminocarbonylphenyl)propylamine. Hydrogenation of the product thus formed according to the procedure of Preparation 3 effected debenzylation to provide S-1-methyl-3-(4-methylaminocarbonylphenyl)propylaminium chloride. $[\alpha]_D$ —6° $[\alpha]_{365}$ —42.3° (MeOH).

Preparation 5

S-1-Methyl-3-(4-aminocarbonylphenyl)propylamine

To 213 g. of (S)-(—)-α-methylbenzylamine in 1 l. of benzene was added 290 g. of 4-(4-aminocarbonylphenyl)-2-butanone and 0.5 g. of *p*-toluenesulfonic acid. The reaction mixture was stirred and heated to reflux. The water that was formed during the reaction was azeotropically removed. After the theoretical amount of water had separated, the benzene was removed at reduced pressure, and the residue was dissolved in methanol and hydrogenated in the presence of Raney nickel at ambient temperature over a sixteen hour period. The catalyst was removed by filtration, and the methanol and any unreacted α-methylbenzylamine were removed at reduced pressure. The residue was dissolved in 500 ml. of ethanol and an excess of hydrogen chloride in ether was added. The solid that formed was filtered, washed with ether, and recrystallized from a mixture of ethanol and acetone. Several more recrystallizations from a mixture of methanol and acetonitrile gave 263 g. of S,S—N-(α-methylbenzyl)-1-3-(4-aminocarbonylphenyl)propylamine, hydrochloride, m.p. 180—182°C. $[\alpha]_D$ −72.2, $[\alpha]_{365}$ −257.5° (MeOH).

A mixture of 258 g. of the above-named hydrochloride salt, 26 g. of 5 percent palladium on carbon and 1700 ml. of ethanol was hydrogenated at 60°C. for eight and one half hours. The catalyst was removed by filtration and the solvent was evaporated. The residue was dissolved in hot ethanol, ether was added and 149 g. of crystalline title product was removed by filtration, m.p. 198—202°C., $[\alpha]_D$ −8.5, $[\alpha]_{365}$ −23.5° (MeOH).

Example 1

R,S—N-(2-Phenyl-2-hydroxyethyl)-1-methyl-3-(4-methylaminocarbonylphenyl)propylamine

To a refluxing solution of 3.6 g. of S-1-methyl-3-(4-methylaminocarbonylphenyl)propylamine in 50 ml. of ethanol was added dropwise over ten minutes a solution of 2.1 g. of R-styrene oxide in 20 ml. of ethanol. Following complete addition of the styrene oxide, the reaction mixture was heated at reflux for four hours and then cooled to 25°C. and stirred at that temperature for twelve hours. The solvent was next removed by evaporation under reduced pressure to provide an oil which was then crystallized from diethyl ether to provide 3.5 g. of the product as a solid. The solid was recrystallized from ethyl acetate and diethyl ether to afford 1.7 g. of R,S—N-(2-phenyl-2-hydroxyethyl)-1-methyl-3-(4-methylaminocarbonylphenyl)propylamine. M.P. 158—160°C.

The amine so formed was converted to the hydrochloride salt by reaction with hydrogen chloride in diethyl ether to provide, following crystallization from methanol and ethyl acetate, 1.2 g. of R,S—N-(2-phenyl-2-hydroxyethyl)-1-methyl-3-(4-methylaminocarbonylphenyl)propylaminium chloride. M.P. 179—182°C.

Analysis calc. for $C_{20}H_{27}ClN_2O_2$

Theory: C, 66.19; H, 7.50; N, 7.72.

Found: C, 66.40; H, 7.82; N, 7.49.

Example 2

R,S—N-(2-Phenyl-2-hydroxyethyl)-1-ethyl-3-(4-hydroxyphenyl)propylamine

A solution of 7.16 g. of (S)-1-ethyl-3-(4-hydroxyphenyl)propylamine in 50 ml. of N,N-dimethylformamide (DMF) was cooled in an ice-water bath and stirred under a nitrogen gas atmosphere while a solution of 6.08 g. of R-mandelic acid and 5.41 g. of 1-hydroxybenzotriazole in 15 ml. of DMF was added in one portion. A solution of 8.24 g. of dicyclohexylcarbodiimide in 10 ml. of DMF was added dropwise over thirty minutes to the stirred reaction mixture. The reaction mixture was stirred for two hours at 5°C., and then allowed to set for twelve hours at 0°C. Dicyclohexylurea had precipitated out of solution, and was separated from the reaction mixture by filtration. Removal of the solvent from the filtrate by evaporation under reduced pressure provided a solid residue. The solid was purified by washing it with sodium carbonate followed by crystallization from acetonitrile to provide 7.31 g. of R,S—N-(2-phenyl-2-hydroxy-1-oxoethyl)-1-ethyl-3-(4-hydroxyphenyl)propylamine. M.P. 116—118.5°C. $[\alpha]_D$ −61° (MeOH).

Analysis calc. for $C_{19}H_{23}NO_3$

Theory: C, 72.82; H, 7.40; N, 4.47.

Found: C, 72.73; H, 7.22; N, 4.77.

A solution of 6.17 g. of the amide thus formed in 70 ml. of tetrahydrofuran (THF) was added dropwise over one hour to a stirred solution of 60 ml. of 1 molar diborane in THF. Following the addition, the reaction mixture was stirred at 24°C. under a nitrogen gas atmosphere for forty-eight hours. Excess methanol then was added to the reaction mixture in order to decompose any unreacted diborane. The solvents were removed from the reaction mixture by evaporation under reduced pressure to provide the product as an oil. The oil so formed was dissolved in ethyl alcohol and diethyl ether, and excess hydrogen chloride gas was bubbled through the reaction mixture in order to form the hydrogen chloride acid addition salt. The salt precipitated out of solution and was removed therefrom by filtration. The solid product so formed was recrystallized several times from ethyl acetate to afford 171 mg. of R,S,—N-(2-phenyl-2-hydroxyethyl)-1-ethyl-3-(4-hydroxyphenyl)propylaminium chloride. M.P. 148—151°C. $[\alpha]_D$ −25.0° (MeOH).

Analysis calc. for $C_{19}H_{26}ClNO_2$
Theory: C, 67.94; H, 7.80; H, 4.17.
Found: C, 67.97; H, 7.72; N, 4.45.

Example 3

R,S—N-[2-(2-Fluorophenyl)-2-hydroxyethyl]-1-methyl-3-(4-hydroxyphenyl)propylamine

A solution of 15.05 g. of S-1-methyl-3-(4-benzyloxyphenyl)propylamine in 50 ml. of DMF was cooled in an ice-water bath and stirred under a nitrogen gas atmosphere while a solution of 10.0 g. of R-2-(2-fluorophenyl-2-hydroxy)acetic acid and 7.97 g. of 1-hydroxybenzotriazole in 50 ml. of DMF was added in one portion. the reaction mixture was stirred at 5°C. while a solution of 12.15 g. of dicyclohexylcarbodiimide in 20 ml. of DMF was added dropwise over thirty minutes. The reaction mixture was stirred at 5°C. for one hour, and then allowed to stand at 9°C. for twelve hours. Dicyclohexylurea had precipitated out of solution, and was removed from the reaction mixture by filtration. The filtrate was diluted with ethyl acetate, washed with sodium carbonate, and then concentrated by evaporation of the solvent under reduced pressure, thus providing 23.0 g. of R,S—N-[2-(2-fluorophenyl)-2-hydroxy-1-oxoethyl]-1-methyl-3-(4-benzyloxyphenyl)propylamine as an oil.

To a stirred solution of 15.2 g. of lithium aluminum hydride in 800 ml. of diethyl ether was added dropwise over one hour a solution of 23.0 g. of the amide thus formed in 100 ml. of THF. After the addition was complete, the reaction mixture was heated at reflux for six hours, and then stirred for an additional twelve hours at 24°C. Any unreacted lithium aluminum hydride remaining in the reaction mixture was decomposed by the dropwise addition of 16 ml. of water, followed by the addition of 12 ml. of twenty percent aqueous sodium hydroxide solution and 56 ml. of water. Filtration and removal of the solvents from the reaction mixture by evaporation under reduced pressure provided R,S—N-[2-(2-fluorophenyl-2-hydroxyethyl]-1-methyl-3-(4-benzyloxyphenyl)propylamine. The residue so formed was dissolved in ethyl alcohol and diethyl ether, and excess hydrogen chloride gas was bubbled through the solution. The precipitate which formed was collected by filtration and recrystallized twice from ethyl alcohol and diethyl ether, affording 10.0 g. of R,S—N-[2-(2-fluorophenyl)-2-hydroxyethyl]-1-methyl-3-(4-benzyloxyphenyl)propylaminium chloride. M.P. 162—163°C.

Analysis calc. for $C_{25}H_{29}ClFNO_2$
Theory: C, 69.84; H, 6.80; N, 3.26.
Found: C, 70.04; H, 6.95; N, 3.41.

A solution of 10.0 g. of the salt thus formed in 190 ml. of ethyl alcohol containing 2.0 g. of a suspension containing five percent of palladium on carbon was heated at 50°C. and stirred for two hours under a hydrogen gas atmosphere at 50 p.s.i. The reaction mixture was then cooled to room temperature and filtered. The filtrate was concentrated by evaporation of the solvent under reduced pressure, thus affording, after crystallization twice from ethanol and ether, 6.9 g. of R,S—N-[2-(2-fluorophenyl)-2-hydroxyethyl]-1-methyl-3-(4-hydroxyphenyl)propylaminium chloride. M.P. 180—183°C. $[\alpha]_D$ —40.6° (MeOH).

Analysis calc. for $C_{18}H_{23}ClFNO_2$
Theory: C, 63.62; H, 6.82; Cl, 10.43;
F, 5.54; N, 4.12.
Found: C, 63.44; H, 6.81; Cl, 10.63;
F, 5.38; N, 4.27.

Example 4

R,S—N-[2-(2-Fluorophenyl)-2-hydroxyethyl]-1-ethyl-3-(4-hydroxyphenyl)propylamine

A solution of 11.62 g. of S-1-ethyl-3-(4-hydroxyphenyl)propylamine in 50 ml. of DMF was cooled in an ice-water bath and stirred under a nitrogen gas atmosphere while to it was added in one portion a solution of 11.0 g. of R-2-(2-fluorophenyl-2-hydroxyl)acetic acid in 20 ml. DMF containing 8.78 g. of 1-hydroxybenzotriazole. The reaction mixture was stirred at 5°C. while a solution of 13.39 g. of dicyclohexylcarbodiimide in 15 ml. of DMF was added dropwise over thirty minutes. Following complete addition of the dicyclohexylcarbodiimide solution, the reaction mixture was stirred at 5°C. for two hours, and then was stored at 0°C. for an additional seventy-two hours. Dicyclohexylurea precipitated out of solution and was removed from the reaction mixture by filtration. The filtrate was concentrated by evaporation of the solvent under reduced pressure to provide an oil. The oil was dissolved in 200 ml. of ethyl acetate, and washed with dilute aqueous hydrochloric acid solution, with aqueous sodium carbonate solution, and with water. The solution was dried and the solvent was removed therefrom by evaporation under reduced pressure to provide R,S—N-[2-(2-fluorophenyl)-2-hydroxy-1-oxoethyl]-1-ethyl-3-(4-hydroxyphenyl)propylamine as a solid. M.P. 114—118°C.

A solution of 13.0 g. of the above-named amide derivative in 100 ml. of tetrahydrofuran (THF) was added dropwise over one hour to a stirred solution of 105 ml. of 1 molar diborane in THF. Following complete addition of the amide derivative, the reaction mixture was stirred for six hours at 24°C., and then was cooled to 0°C. and allowed to set for twelve hours. The reaction mixture was next warmed to room temperature and stirred while excess methanol was added to decompose any

unreacted diborane remaining in the reaction mixture. Evaporation of the reaction solvent under reduced pressure then provided R,S—N-[2-(2-fluorophenyl)-2-hydroxyethyl]-1-ethyl-3-(4-hydroxyphenyl)propylamine as an oil. The oil so formed was dissolved in ethanol, and diethyl ether containing excess hydrogen chloride gas was added. The hydrochloride salt of the above-named amine precipitated out of solution, and was recovered by filtration. Recrystallization of the solid so formed from ethanol and diethyl ether afforded R,S—N-[2-(2-fluorophenyl)-2-hydroxyethyl]-1-ethyl-3-(4-hydroxyphenyl)propylaminium chloride. M.P. 121—124°C. $[\alpha]_D$ −27.3° (MeOH).

Analysis calc. for $C_{19}H_{25}ClFNO_2$

Theory: C, 64.49; H, 7.12; Cl, 10.02; F, 5.37; N, 3.96.

Found: C, 64.58; H, 6.91; Cl, 10.16; F, 5.47; N, 4.11.

Example 5

R,S—N-(2-Phenyl-2-hydroxyethyl)-1-methyl-3-(4-hydroxyphenyl)propylamine

A solution of 122.0 g of S-1-methyl-3-(4-hydroxyphenyl)propylamine in 300 ml. of DMF was cooled to 5°C. in an ice-water bath and stirred while a solution of 112.6 g. of R-mandelic acid and 100.0 g. of 1-hydroxybenzotriazole in 300 ml. of DMF was added in one portion. The reaction mixture was stirred at 5°C. while a solution of 156.0 g. of dicyclohexylcarbodiimide in 500 ml. of DMF was added dropwise over two hours. Following the complete addition of the dicyclohexylcarbodiimide, the reaction mixture was stirred for an additional fifteen minutes, and then was allowed to set at 0°C. for twelve hours. The reaction mixture then was filtered to remove the precipitated dicyclohexylurea. Next, the solvent was removed from the reaction mixture by evaporation under reduced pressure, leaving an oily residue. The oil so formed was dissolved in 1000 ml. of ethyl acetate and washed with aqueous sodium carbonate and water. After drying the solution, the solvent was removed by evaporation under reduced pressure, thus providing 205.8 g of R,S—N-(2-phenyl-2-hydroxy-1-oxoethyl)-1-methyl-3-(4-hydroxyphenyl)propylamine. The amide thus formed was recrystallized from ethyl acetate and hexane. M.P. 219—223°C. $[\alpha]_D$ −54.1° (MeOH).

Analysis calc. for $C_{18}H_{21}NO_3$

Theory: C, 72.22; H, 7.07; N, 4.68.

Found: C, 71.98; H, 6.79; N, 8.89.

A solution of 168.2 g. of the amide and 170 ml. of triethylamine in 3000 ml. of benzene and 700 ml. of THF was stirred at 24°C. while 143 ml. of trimethylchlorosilane was added portionwise. The reaction mixture was stirred for thirty minutes and then filtered to remove the precipitated triethylamine hydrochloride. The filtrate was concentrated by evaporation of the solvent under reduced pressure, leaving an oily residue. The oil was next dissolved in 1000 ml. of THF, and the solution was stirred at 24°C. under a nitrogen gas atmosphere while 1300 ml. of 1 molar diborane in THF was added portionwise. The reaction mixture was stirred at room temperature for sixteen hours, and then excess methanol was added to the solution in order to decompose any unreacted diborane and to hydrolyze the trimethylsilyl ester. Removal of the solvent by evaporation under reduced pressure provided an oil. The oil was dissolved in diethyl ether containing hydrogen chloride, and the salt which formed was collected by filtration and recrystallized from ethanol and diethyl ether, affording 131.0 g. of R,S—N-(2-phenyl-2-hydroxyethyl)-1-methyl-3-(4-hydroxyphenyl)propylaminium chloride. M.P. 149—150.5°C. $[\alpha]_D$ −51.7° (MeOH).

Example 6

R,S—N-(2-Phenyl-2-hydroxyethyl)-1-methyl-3-(4-hydroxyphenyl)propylamine

A solution of 10.4 g. of S-1-methyl-3-(4-benzyloxyphenyl)propylamine in 150 ml. of DMF was stirred at room temperature under a nitrogen gas atmosphere while 6.35 g. of R-mandelic acid and 5.63 g. of 1-hydroxybenzotriazole were added. The reaction mixture was cooled to −5°C. in an ice and methanol bath and stirred while a solution of 9.03 g. of dicyclohexylcarbodiimide in 70 ml. of DMF was added dropwise over ten minutes. The reaction mixture was then stirred at 5°C. for one hour and then stored at 0°C. for twelve hours. The reaction mixture next was filtered, thus removing the precipitated dicyclohexylurea. The filtrate was concentrated by evaporation of the solvent under reduced pressure to provide an oily residue. The oil so formed was dissolved in 400 ml. of benzene and washed with aqueous sodium carbonate solution, with 1 N hydrochloric acid solution, and with water. After drying the reaction mixture, the solvent was removed by evaporation under reduced pressure, providing 13.6 g. of R,S—N-(2-phenyl-2-hydroxy-1-oxoethyl)-1-methyl-3-(4-benzyloxyphenyl)propylamine as a crystalline solid. M.P. 102—104.5°C. $[\alpha]_D$ −48.98° (MeOH).

Analysis calc. for $C_{25}H_{27}NO_3$

Theory: C, 77.09; H, 6.99; N, 3.60.

Found: C, 76.87; H, 7.03; N, 3.65.

A solution of 110 ml. of 1 molar diborane in THF was diluted with an additional 100 ml. of THF. The solution was cooled in an ice-acetone bath and stirred under a nitrogen gas atmosphere

while a solution of 12.3 g. of R,S—N-(2-phenyl-2-hydroxy-1-oxoethyl)-1-methyl-3-(4-benzyloxyphenyl)propylamine in 75 ml. of THF was added dropwise over twenty minutes. The reaction mixture was heated at reflux for four hours, and then cooled to room temperature and stirred for an additional twelve hours. Excess methanol next was added to the reaction mixture in order to decompose any unreacted diborane. Concentration of the reaction mixture by evaporation of the solvent under reduced pressure provided an oily residue. The oil was dissolved in 100 ml. of methanol and 100 ml. of diethyl ether. The solution was stirred and hydrogen chloride was bubbled through the solution, thus forming a precipitate. The precipitate was collected by filtration and recrystallized from acetonitrile. The crystalline solid so formed was suspended in ethyl acetate and washed with sodium carbonate and water. After drying the ethyl acetate solution, the solvent was removed by evaporation under reduced pressure, thus affording R,S—N-(2-phenyl-2-hydroxyethyl)-1-methyl-3-(4-benzyloxyphenyl)propylamine. M.P. 113—115.5°C. $[\alpha]_D$ −17.5° (MeOH).

Analysis calc. for $C_{25}H_{29}NO_2$

Theory: C, 79.96; H, 7.78; N, 3.73.

Found: C, 79.74; H, 7.82; N, 3.43.

A solution of 5.9 g. of the amine thus formed in 50 ml. of methanol was stirred at 24°C. while a solution of excess hydrogen chloride gas in diethyl ether was added in one portion. The precipitated solid which immediately formed was collected by filtration and then was recrystallized from acetonitrile and diethyl ether, providing 6.39 g. of the amine as the hydrochloride salt. M.P. 158.0—160.5°C. $[\alpha]_D$ −36.1 (MeOH).

Analysis calc. for $C_{25}H_{30}ClNO_2$

Theory: C, 72.89; H, 7.34; N, 3.40.

Found: C, 72.91; H, 7.34; N, 3.27.

A solution of 10.6 g. of R,S—N-(2-phenyl-2-hydroxyethyl)-1-methyl-3-(4-benzyloxyphenyl)propylaminium chloride in 140 ml. of methanol was stirred while 1.0 g. of a five percent suspension of palladium on carbon was added portionwise. The reaction mixture then was stirred for four hours at 60°C. under a hydrogen gas atmosphere at 50 p.s.i. The reaction mixture then was filtered, and the filtrate was concentrated by evaporation of the solvent under reduced pressure, thus providing 10.6 g. of a pink foam. The foam was dissolved in 600 ml. of water and stirred while a solution of 35 g. of sodium carbonate in 300 ml. of water was added portionwise over ten minutes. The product precipitated out of the aqueous alkaline solution and was collected by filtration. Recrystallization of the solid product so formed twice from 450 ml. of hot ethyl acetate provided 6.63 g. of R,S—N-(2-phenyl-2-hydroxyethyl)-1-methyl-3-(4-hydroxyphenyl)propylamine. M.P. 169.5—173°C. $[\alpha]_D$ −24.5° (MeOH).

Analysis calc. for $C_{18}H_{23}NO_2$

Theory: C, 75.76; H, 8.12; N, 4.91.

Found: C, 75.99; H, 8.11; N, 4.68.

Example 7

R,S—N-(2-Phenyl-2-hydroxyethyl)-1-methyl-3-(4-hydroxyphenyl)propylaminium chloride

A solution of 6.63 g. of the free amine from Example 6 in 200 ml. of methanol was stirred while a solution of hydrogen chloride in diethyl ether was added in one portion. The solvent was removed by evaporation under reduced pressure to provide the product as a white solid. The solid was collected by filtration and then was recrystallized from acetone and diethyl ether to afford R,S—N-(2-phenyl-2-hydroxyethyl)-1-methyl-3-(4-hydroxyphenyl)propylaminium chloride. M.P. 150—159°C. $[\alpha]_D$ −47.0° (MeOH).

Analysis calc. for $C_{18}H_{24}ClNO_2$

Theory: C, 67.17; H, 7.52; N, 4.35; Cl, 11.02.

Found: C, 66.97; H, 7.29; N, 4.50; Cl, 11.32.

Example 8

R,S—N-(2-Phenyl-2-hydroxyethyl)-1-methyl-3-(4-acetoxyphenyl)propylamine

A solution of 6.03 g. of R,S—N-(2-phenyl-2-hydroxyethyl)-1-methyl-3-(4-hydroxyphenyl)propylamine dissolved in 350 ml. of tetrahydrofuran and 200 ml. of benzene was stirred at room temperature while 9.05 g. of triethylamine was added in one portio· The reaction mixture was stirred while a solution of 4.97 g. of thionyl chloride in 100 ml. of benzene was added dropwise over eight minutes. An additional 3.62 g. of triethylamine dissolved in 50 ml. of benzene was added to the reaction mixture, and then the reaction mixture was stirred for two hours at room temperature. The reaction mixture was washed several times with aqueous saturated sodium bicarbonate solution, and then with water. The solvent was removed from the reaction mixture by evaporation under reduced pressure to provide 8.1 g. of R,S—N-[1-methyll-3-(4-hydroxyphenyl)propyl]-5-phenyl-1-oxo-4,5-dihydro-1,2,3-oxathiazole as an orange oil.

A solution of 3.6 g. of the above-named oil in 20 ml. of benzene was stirred at room temperature while 1.5 ml. of pyridine was added, followed by the addition to the reaction mixture of 1.15 ml. of acetic anhydride. The reaction mixture was refluxed and stirred for one hour. The reaction mixture was cooled to room temperature and allowed to stand for twelve hours. The reaction mixture was next poured into 50 ml. of xylene, and the solvents were then removed by evaporation under reduced pressure to provide an oil. The oil was chromatographed over 25 g. of alumina, eluting with benzene and 80% benzene in ethyl acetate. Collection of the appropriate fractions and evaporation of the solvent therefrom gave 2.41 g. of an oil. A solution of 2.41 g of the oil in 50 ml. of ethyl alcohol containing 4.5 ml. of 3 N hydrochloric acid was stirred for fifteen minutes at room temperature. The reaction mixture was then poured into 50 ml. of xylene, and the solvents were removed by evaporation under reduced pressure, providing 2.17 g. of a solid residue. Crystallization from ethyl acetate of the solid residue thus formed provided 1.77 g. of R,S—N-(2-phenyl-2-hydroxyethyl)-1-methyl-3-(4-acetoxyphenyl)propylaminium chloride. M.P. 160—161°C. $[\alpha]_D$ −44° (MeOH).

Analysis calc. for $C_{20}H_{26}ClNO_3$

| | | | |
|---|---|---|---|
| Theory: | C, 66.02; | H, 7.20; | N, 3.85; |
| | Cl, 9.74. | | |
| Found: | C, 65.92; | H, 7.31; | N, 3.86; |
| | Cl, 9.78. | | |

## Example 9

### R,S—N-(2-Phenyl-2-hydroxyethyl)-1-methyl-3-(4-isobutyroxyphenyl)propylamine

A solution of 4.5 g. of R,S—N-[1-methyl-3-(4-hydroxyphenyl)propyl]-5-phenyl-1-oxo-4,5-dihydro-1,2,3-oxathiazole in 40 ml. of benzene was stirred at room temperature while 4.0 ml. of pyridine and 2.85 g. of isobutyric anhydride were added in one portion. The reaction mixture was stirred and heated at reflux for sixteen hours. The reaction mixture was cooled and diluted with xylene, and the solvents were removed by evaporation under reduced pressure, providing 4.36 g. of an oil. The oil so formed was dissolved in 8 ml. of 3 N hydrochloric acid, and the mixture was stirred for fifteen minutes at room temperature. The reaction mixture was next poured into 50 ml. of xylene, and concentrated to dryness under reduced pressure, providing 4.34 g. of a solid residue. The solid thus formed was crystallized from acetonitrile to afford 3.75 g. of R,S—N-(2-phenyl-2-hydroxyethyl)-1-methyl-3-(4-isobutyroxyphenyl)-propylaminium chloride. M.P. 158.5—160°C. $[\alpha]_D$ −41.6° (MeOH).

Analysis calc. for $C_{22}H_{30}ClNO_3$

| | | | |
|---|---|---|---|
| Theory: | C, 67.42; | H. 7.72; | N, 3.57; |
| | Cl, 9.05. | | |
| Found: | C, 67.61; | H, 8.01; | N, 3.74; |
| | Cl, 9.16. | | |

## Example 10

### R,S—N-(2-Phenyl-2-hydroxyethyl)-1-methyl-3-(4-methoxycarbonylphenyl)propylamine

Methyl 2-(4-hydroxycarbonylphenyl)ethyl ketone was esterified by reaction with methyl alcohol and hydrochloric acid to provide methyl 2-(4-methoxycarbonylphenyl)ethyl ketone. The ketone was condensed with optically active S-$\alpha$-methylbenzylamine, and the imine which was formed was reduced to provide N-($\alpha$-methylbenzyl)-1-methyl-3-(4-methoxycarbonylphenyl)propylamine. The amine was converted to the hydrochloride salt, and fractional crystallization of the salt thus formed afforded optically active S,S—N-($\alpha$-methylbenzyl)-1-methyl-3-(4-methoxycarbonylphenyl)propylaminium chloride. M.P. 198—205°C. $[\alpha]_D$ −81.2° (MeOH).

The resolved $\alpha$-methylbenzyl protected aminium chloride was hydrogenated in the presence of Raney nickel to effect removal of the $\alpha$-methylbenzyl protecting group, thus providing optically active S-1-methyl-3-(4-methoxycarbonylphenyl)propylaminium chloride. M.P. 165—172°C. $[\alpha]_D$ −6.7° (MeOH).

A solution of S-1-methyl-3-(4-methoxycarbonylphenyl)propylaminium chloride in ethyl acetate was reacted with aqueous sodium carbonate to provide 4.8 g. of S-1-methyl-3-(4-methoxycarbonylphenyl)propylamine. The optically active free amine was dissolved in a solution of 150 ml. of N,N-dimethylformamide containing 3.52 g. of R-mandelic acid and 3.4 g. of 1-hydroxybenzothiazole. The reaction mixture was stirred while a solution of 4.78 g. of N,N′-dicyclohexylcarbodiimide in 50 ml. of DMF was added dropwise over twenty minutes. Following the complete addition of the dicyclohexylcarbodiimide, the reaction mixture was cooled to about 10°c. and stored for twelve hours at that temperature. The dicyclohexylurea which had precipitated out of the reaction solution was removed by filtration, and the filtrate was concentrated to dryness by evaporation of the solvent under reduced pressure to provide an oil. The oil so formed was dissolved in ethyl acetate and washed with aqueous sodium carbonate, with water, and dried. Evaporation of the solvent under reduced pressure afforded an oil which was crystallized from diethyl ether and hexane to provide 6.27 g. of R,S-(2-phenyl-2-hydroxy-1-oxoethyl)-1-methyl-3-(4-methoxycarbonylphenyl)propylamine. M.P. 99—101°C. $[\alpha]_D$ −59.2° (MeOH).

Analysis calc. for $C_{20}H_{23}NO_4$
Theory: C, 70.36; H, 6.79; N, 4.10.
Found: C, 70.57; H, 6.85; N, 4.21.

The above reactions were repeated on several occasions to provide additional quantities of the amide. To a stirred solution of 26.2 g. of R,S—N-(2-phenyl-2-hydroxy-1-oxoethyl)-1-methyl-3-(4-methoxycarbonylphenyl)propylamine in 300 ml. of tetrahydrofuran was added portionwise over one hour 400 ml. of a 1.02 N solution of diborane in tetrahydrofuran. Following the addition, the reaction mixture was stirred at 25°C. for twenty-six hours. The reaction mixture next was diluted by the addition of 250 ml. of methanol. Removal of the reaction solvent by evaporation under reduced pressure provided the product as a crude oil. The oil was dissolved in 50 ml. of fresh methanol, and the solution was diluted with 100 ml. of diethyl ether saturated with hydrogen chloride, thereby forming the hydrochloride salt of the product as a white solid. The solid was recrystallized from 100 ml. of acetonitrile to provide 15.60 g. of R,S—N-(2-phenyl-2-hydroxyethyl)-1-methyl-3-(4-methoxycarbonylphenyl)propylaminium chloride. M.P. 141—155°C. The salt was converted to the free amine base which was crystallized from ethyl acetate. M.P. 125—128°C. The free base was converted to the hydrochloride salt, which when crystallized from acetonitrile and diethyl ether afforded the desired product having a M.P. 149—154°C. $[\alpha]_D$ − 48.8° (MeOH).

Analysis calc. for $C_{20}H_{26}ClNO_3$
Theory: C, 66.02; H, 7.20; N, 3.85.
Found: C, 65.91; H, 6.96; N, 3.78.

## Example 11

### R,S—N-(2-Phenyl-2-hydroxyethyl)-1-methyl-3-(4-aminocarbonylphenyl)propylamine

A solution of 1.21 g. of R,S—N-(2-phenyl-2-hydroxyethyl)-1-methyl-3-(4-methoxycarbonylphenyl)propylamine in 60 ml. of ethyl alcohol containing 20 ml. of anhydrous hydrazine was stirred and heated at reflux for twenty-eight hours. The reaction mixture then was cooled to room temperature and the solvent was removed therefrom by evaporation under reduced pressure to provide a product which was crystallized from isopropanol to give 940 mg. of R,S—N-(2-phenyl-2-hydroxyethyl)-1-methyl-3-(4-hydrazinocarbonylphenyl)propylamine. M.P. 134—142°C. $[\alpha]_D$ −27.9° (MeOH).

Analysis calc. for $C_{19}H_{25}N_3O_2$
Theory: C, 69.70; H, 7.70; N, 12.83.
Found: C, 69.72; H, 7.44; N, 12.97.

A solution of 720 mg. of R,S—N-(2-phenyl-2-hydroxyethyl)-1-methyl-3-(4-hydrazinocarbonylphenyl)propylamine in 100 ml. of ethanol containing 8 g. of Raney nickel was hydrogenated in a Brown hydrogenation apparatus utilizing 4 g. of sodium borohydride as the hydrogen source. The reaction mixture was stirred at 25°C. for twelve hours, and then was filtered through hyflo super cell. The filtrate was evaporated to dryness under reduced pressure to provide a solid. The solid residue thus formed was dissolved in 50 ml. of water containing 25 ml. of 3 N hydrochloric acid. The aqueous acid solution was washed with ethyl acetate, and then made basic by the gradual addition of ammonium hydroxide. The aqueous alkaline solution was extracted several times with fresh portions of ethyl acetate. The organic extracts were combined, washed with water and dried. Removal of the solvent by evaporation under reduced pressure provided, as a white solid, R,S—N-(2-phenyl-2-hydroxyethyl)-1-methyl-3-(4-aminocarbonylphenyl)propylamine. The solid thus formed was dissolved in methanol and the solution was diluted with 30 ml. of diethyl ether saturated with hydrogen chloride. Filtration of the precipitated solid which formed afforded 72.6 mg. of R,S—N-(2-phenyl-2-hydroxyethyl)-1-methyl-3-(4-aminocarbonylphenyl)propylaminium chloride. M.P. 229—230°C. $[\alpha]_D$ −51.6° (MeOH).

Analysis calc. for $C_{19}H_{25}ClN_2O_2$
Theory: C, 65.41; H, 7.22; N, 8.03;
Cl, 10.16.
Found: C, 65.29; H, 7.14; N, 8.24;
Cl, 10.04.

## Example 12

### R,S—N-(2-Phenyl-2-hydroxyethyl)-1-methyl-3-(4-aminocarbonylphenyl)propylaminium chloride

To a refluxing solution of 109.6 g. of S-1-methyl-3-(4-aminocarbonylphenyl)propylamine in 600 ml. of ethanol and 200 ml. of methanol was added dropwise over one hour a solution of 68.4 g. of R-styrene oxide in 100 ml. of ethanol. The reaction mixture was heated at reflux for three hours following complete addition of the styrene oxide, and then was cooled to room temperature and concentrated by evaporation of the solvent under reduced pressure. The reaction mixture next was diluted by the addition of 200 ml. of diethyl ether and filtered. The precipitate was triturated with water, dried, and recrystallized from acetonitrile to provide 72.6 g. of the free amine product as a solid. The amine so formed was converted to the hydrochloride salt by reaction with hydrogen chloride in diethyl ether to provide 64.0 g. of R,S—N-(2-phenyl-2-hydroxyethyl)-1-methyl-3-(4-aminocarbonylphenyl)propyl-

aluminium chloride. M.P. 229—231°C. $[\alpha]_D$ −52.1° (MeOH).

Analysis calc. for $C_{19}H_{25}ClN_2O_2$

Theory: C, 65.41; H, 7.22; N, 8.03.

Found: C, 65.17; H, 7.10; N, 8.03.

Example 13

R,S—N-(2-Phenyl-2-hydroxyethyl)-1-methyl-3-(4-hydroxyphenyl)propylamine

A mixture of 100 g. of R-mandelic acid and 244 ml. of dichloroacetyl chloride was heated at 60°C. and stirred for three hours. The mixture then was cooled to room temperature and concentrated in volume under reduced pressure to provide a yellow oil. The oil was dissolved in 200 ml. of thionyl chloride, and the reaction mixture was heated to reflux and stirred for four hours. The reaction mixture was cooled to room temperature and excess thionyl chloride was removed by evaporation under reduced pressure. The product was purified by distillation to provide 180.9 g. of R-(2-phenyl-2-dichloroacetoxy)acetyl chloride. B.P. 112.5° 10.67 N/m² (0.08 torr); $[\alpha]_D$ −191° (toluene).

A solution of 4.3 g. of S-1-methyl-3-(4-benzyloxyphenyl)propylamine in 70 ml. of dichloromethane was added in one portion to a stirred suspension of 5.5 g. of sodium bicarbonate and 5.02 g. of R-(2-phenyl-2-dichloroacetoxy)acetyl chloride in 100 ml. of dichloromethane. The reaction mixture was stirred at room temperature for sixteen hours and then filtered. The filtrate was concentrated by evaporation of the solvent under reduced pressure to provide a solid residue. The residue was crystallized from 75 ml. of diethyl ether to afford 6.70 g. of R,S—N-(2-phenyl-2-dichloroacetoxy-1-oxoethyl)-1-methyl-3-(4-benzyloxyphenyl)propylamine. M.P. 110—11.5°C. $[\alpha]_D$ −41.7°C. (MeOH).

Analysis calc. for $C_{27}H_{27}Cl_2NO_4$

Theory: C, 64.80; H, 5.44; N, 2.80; Cl, 14.17.

Found: C, 64.66; H, 5.61; N, 2.94; Cl, 14.42.

A solution of 6.70 g. of the amide thus formed in 130 ml. of tetrahydrofuran containing 50 ml. of 1 N diborane in tetrahydrofuran was heated to reflux and stirred for twenty hours. The reaction mixture was cooled to 60°C. and decomposed by the dropwise addition of 2 ml. of water and 35 ml. of 3 N hydrochloric acid. The aqueous acidic mixture was heated to reflux for two hours, again cooled to about 40°C., and then made alkaline by the addition of 100 ml. of 5 N sodium hydroxide. After stirring the alkaline mixture for two hours at room temperature, the organic layer was separated and the aqueous alkaline layer was extracted with ethyl acetate. The organic layers were combined, washed with brine, dried and the solvent was evaporated to provide 5.1 g. of a solid residue. The solid was dissolved in 100 ml. of hot ethanol and acidified by the addition of a diethyl ether solution of hydrogen chloride. The crystalline precipitate which formed was collected by filtration and dried to provide 4.40 g. of R,S—N-(2-phenyl-2-hydroxyethyl)-1-methyl-3-(4-benzyloxyphenyl)propylaminium chloride. M.P. 160—162.5°C. $[\alpha]_D$ −37.8° (MeOH).

Analysis calc. for $C_{25}H_{30}ClNO_2$

Theory: C, 72.89; H, 7.34; N, 3.40; Cl, 8.61.

Found: C, 73.02; H, 7.58; N, 3.64; Cl, 8.96.

Hydrogenolysis of 4.28 g. of the amine salt thus formed in the presence of 500 mg. of five percent palladium on carbon in methanol for three hours at 40°C. effected removal of the benzyl protecting group to provide, after purification by crystallization from acetone and diethyl ether, 2.79 g. of R,S—N-(2-phenyl-2-hydroxyethyl)-1-methyl-3-(4-hydroxyphenyl)propylaminium chloride. M.P. 153—154°C. $[\alpha]_D$ −49.9° (MeOH).

Analysis calc. for $C_{18}H_{24}ClNO_2$

Theory: C, 67.17; H, 7.52; N, 4.35; Cl, 11.02.

Found: C, 67.08; H, 7.33; N, 4.51; Cl, 11.37.

Example 14

Preparation of 250 mg. Tablets

| | |
|---|---|
| R,S—N-(2-phenyl-2-hydroxyethyl)-1-methyl-3-(4-aminocarbonylphenyl)-propylaminium chloride | 250 mg. |
| Lactose | 200 mg. |
| Corn Starch | 300 mg. |

| | |
|---|---|
| Corn Starch Paste | 50 mg. |
| Calcium Stearate | 5 mg. |
| Dicalcium Phosphate | 45 mg. |

The active drug, corn starch, lactose, and dicalcium phosphate are uniformly blended. The corn starch paste is prepared as a 10 percent aqueous paste and is blended into the mixture to uniformity. The mixture is blended with the calcium stearate and then compressed into tablets. Such tablets can be administered to an obese subject at the rate of 1 to about 4 tablets per day or as needed.

Example 15
Preparation of Suppositories

| | |
|---|---|
| R,S—N-(2-phenyl-2-hydroxyethyl)-1-methyl-3-(4-hydroxyphenyl)propylaminium chloride | 500 mg. |
| Theobroma oil | 1500 mg. |

The above ingredients are blended to uniformity at a temperature of about 60°C. and then permitted to cool in a tapered mold. Each suppository will weigh about 2 grams and can be administered to a mature obese subject from 1 to 2 times each day for the reduction of weight, or to an immature obese subject for the control of weight gain.

Example 16
Preparation for Oral Suspension

| | |
|---|---|
| R,S—N-[2-(2-fluorophenyl)-2-hydroxyethyl]-1-methyl-3-(4-hydroxyphenyl)propylaminium acetate | 5000 mg. |
| Sorbitol solution (70% N.F.) | 40 mg. |
| Sodium benzoate | 150 mg. |
| Lactose | 10 mg. |
| Cherry flavor | 50 mg. |
| Distilled water q.s. ad | 100 ml. |

The sorbitol solution is added to 40 ml. of distilled water and the R,S—N-[2-(2-fluorophenyl)-2-hydroxyethyl]-1-methyl-3-(4-hydroxyphenyl)propylaminium acetate is dissolved therein. The lactose, sodium benzoate and flavoring are added and dissolved. The volume of the solution is adjusted to 100 ml. with distilled water. Each ml. of syrup contains 50 mg. of active drug. A mature obese mammal will be administered about 5 to about 20 ml. of syrup each day for the effective loss of weight.

**Claims**

1. An optically active compound of the formula (I)

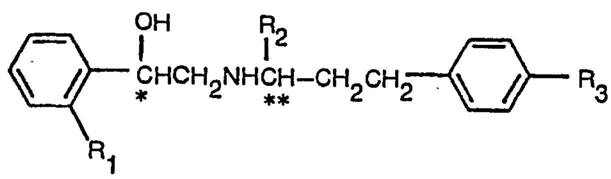

(I)

wherein:

$R_1$ is hydrogen or fluorine;

$R_2$ is methyl or ethyl;

$R_3$ is hydroxy, $C_1$—$C_4$ alkanoyloxy, aminocarbonyl, methylaminocarbonyl or $C_1$—$C_2$ alkoxycarbonyl;

$\underset{*}{C}$ is an asymmetric carbon atom having the R absolute stereochemical configuration; and $\underset{**}{C}$ is an asymmetric carbon atom having the S absolute stereochemical configuration; or a pharmaceutically acceptable acid addition salt thereof.

2. A compound according to Claim 1, wherein $R_1$ is hydrogen.

3. A compound according to Claim 1 or 2, wherein $R_2$ is methyl.

4. A compound according to any one of Claims 1 to 3, wherein $R_3$ is hydroxy or aminocarbonyl.

5. R,S—N-(2-Phenyl-2-hydroxyethyl)-1-methyl-3-(4-aminocarbonyl)propylamine.

6. R,S—N-(2-Phenyl-2-hydroxyethyl)-1-methyl-3-(4-hydroxyphenyl)propylamine.

7. A pharmaceutical formulation comprising a compound of formula (I) as claimed in any one of claims 1 to 6, or a pharmaceutically-acceptable salt acid addition thereof, associated with a pharmaceutically-acceptable carrier therefor.

8. Pharmaceutical formulation as claimed in claim 7, which additionally comprises the S,S-isomer of the R,S-isomer of formula (I) as a diluent.

9. A compound of formula (I) as claimed in any one of claims 1 to 6 or a pharmaceutical formulation as claimed in any one of claims 7 or 8 for use in removing, or preventing the formation of, adipose tissue in mammals.

10. A process for preparing a compound of formula (I) as claimed in any one of claims 1 to 6, which process comprises:

(a) reacting a styrene oxide of formula (II):

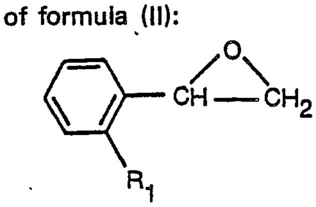

(II)

where $R_1$ is as defined in claim 1 or 2 with a propylamine derivative of formula (IV):

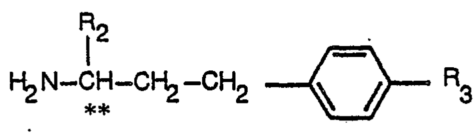

(IV)

where $R_2$ and $R_3$ are as defined in claim 1, 3 or 4; or

(b) reducing a compound of formula (III)

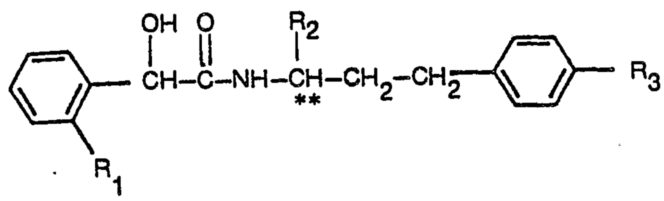

(III)

where $R_1$ and $R_2$ are as defined above and where $R_3$ is also as defined above or benzyloxy, followed in the case where $R_3$ is benzyloxy by hydrogenolysis of the benzyl group to give the compound of formula (I) where $R_3$ is hydroxyl; and

(c) where appropriate, resolving a product of formula (I) which is a mixture of R,S- and S,S-enantiomer.

11. Animal feedstuff comprising a compound of formula (I) as claimed in any one of claims 1 to 6, either alone or associated with the S,S-isomer.

## Revendications

1. Composé optiquement actif répondant à la formule (I):

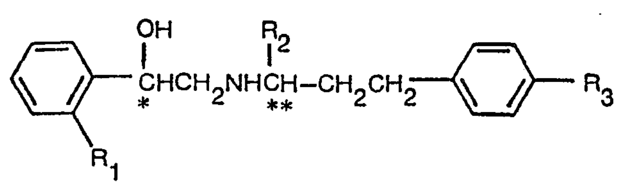

(I)

dans laquelle

$R_1$ représente un atome d'hydrogène ou un atome de fluor;

$R_2$ représente un groupe méthyle ou un groupe éthyle;

$R_3$ représente un groupe hydroxy, un groupe alcanoyl (en $C_1$—$C_4$)oxy, un groupe aminocarbonyle, un groupe méthylaminocarbonyle ou un groupe alcoxy (en $C_1$—$C_2$) carbonyle;

$\underset{*}{C}$ est un atome de carbone asymétrique ayant la configuration stéréochimique absolue R; et

$\underset{**}{C}$ est un atome de carbone asymétrique ayant la configuration stéréochimique absolue S; ou un sel d'addition d'acide pharmaceutiquement acceptable de ce composé.

2. Composé suivant la revendication 1, caractérisé en ce que $R_1$ est un atome d'hydrogène.

3. Composé suivant la revendication 1 ou 2, caractérisé en ce que $R_2$ est un groupe méthyle.

4. Composé suivant l'une quelconque des revendications 1 à 3, caractérisé en ce que $R_2$ est un group hydroxy ou un groupe aminocarbonyle.

5. R,S—N-(2-phényl-2-hydroxyéthyl)-1-méthyl-3-(4-aminocarbonylphényl)propylamine.

6. R,S—N-(2-phényl-2-hydroxyéthyl)-1-méthyl-3-(4-hydroxyphényl)propylamine.

7. Formulation pharmaceutique comprenant un composé de formule (I) suivant l'une quelconque des revendications 1 à 6 ou sel d'addition d'acide pharmaceutiquement acceptable de ce composé, en association avec un support pharmaceutiquement acceptable pour ce composé ou ce sel.

8. Formulation pharmaceutiquement suivant la revendication 7, caractérisée en ce qu'elle comprend, en outre, l'isomère S,S de l'isomère R,S de formule (I) comme diluant.

9. Composé de formule (I) suivant l'une quelconque des revendications 1 à 6 ou une formulation pharmaceutiquement suivant l'une quelconque des revendications 7 et 8 en vue de l'utiliser pour éliminer ou prévenir la formation de tissu adipeux chez les mammifères.

10. Procédé de préparation d'un composé de formule (I) suivant l'une quelconque des revendications 1 à 6, caractérisé en ce qu'il comprend les étapes qui consistent à:

(a) faire réagir un oxyde de styrène de formule (II):

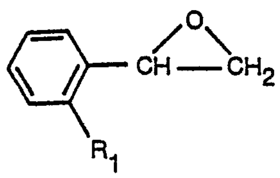

(II)

dans laquelle $R_1$ a la signification définie dans la revendication 1 ou 2,

avec un dérivé de propylamine de formule (IV):

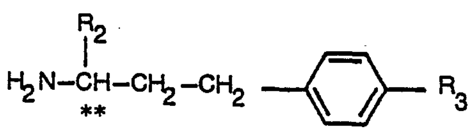

(IV)

dans laquelle $R_2$ et $R_3$ ont les significations définies dans la revendication 1, 3 ou 4; ou

b) réduire un composé de formule (III):

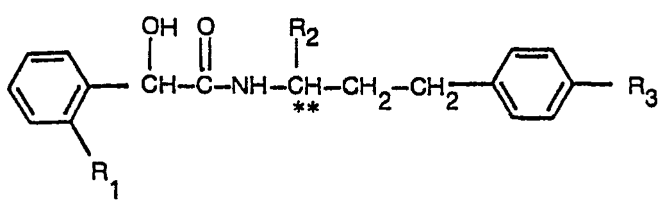

(III)

dans laquelle $R_1$ et $R_2$ ont les significations définies ci-dessus et dans laquelle $R_3$ a également la signification définie ci-dessus ou représente un groupe benzyloxy puis, lorsque $R_3$ est un groupe benzyloxy, effectuer l'hydrogénolyse du groupe benzyle pour obtenir le composé de formule (I) dans laquelle $R_3$ représente un groupe hydroxy; et

(c) lorsque cela est approprié, dédoubler un produit de formule (I) qui est un mélange d'énantiomères R,S et S,S.

11. Aliment pour animaux, caractérisé en ce qu'il comprend un composé de formule (I) suivant l'une quelconque des revendications 1 à 6, seul ou en association avec l'isomère S,S.

**Patentansprüche**

1. Optisch aktive Phenethanolamine der allgemeinen Formel I

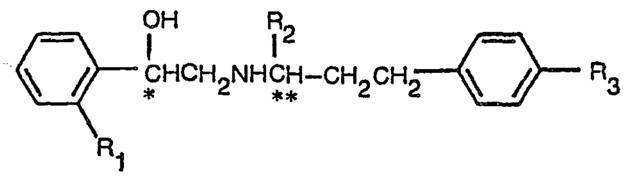

(I)

worin $R_1$ Wasserstoff oder Fluor ist,

$R_2$ für Methyl oder Ethyl steht,

$R_3$ für Hydroxy, $C_1$—$C_4$-Alkanoyloxy, Aminocarbonyl, Methylaminocarbonyl oder $C_1$—$C_2$-Alkoxycarbonyl steht,

$\underset{*}{C}$ ein asymmetrisches Kohlenstoffatomen mit der absoluten stereochemischen Konfiguration R darstellt und

$\underset{**}{C}$ ein asymmetrisches Kohlenstoffatom mit der absoluten stereochemischen Konfiguration S ist,

und die pharmazeutisch unbedenklichen Säureadditionssalze hiervon.

2. Verbindung nach Anspruch 1, dadurch gekennzeichnet, daß $R_1$ Wasserstoff ist.

3. Verbindung nach Anspruch 1 oder 2, dadurch gekennzeichnet, daß $R_2$ Methyl bedeutet.

4. Verbindung nach einem der Ansprüche 1 bis 3, dadurch gekennzeichnet, daß $R_3$ Hydroxy oder Aminocarbonyl ist.

5. R,S—N-(2-Phenyl-2-hydroxyethyl)-1-methyl-3-(4-aminocarbonylphenyl)propylamin.

6. R,S—N-(2-Phenyl-2-hydroxyethyl)-1-methyl-3-(4-hydroxyphenyl)propylamin.

7. Pharmazeutische Formulierung aus einem pharmazeutisch unbedenklichen Träger und einem Wirkstoff, dadurch gekennzeichnet, daß sie als Wirkstoff ein Phenethanolamin der allgemeinen Formel I gemäß einem der Ansprüche 1 bis 6 oder ein pharmazeutisch unbedenkliches Säureadditionssalze hiervon enthält.

8. Pharmazeutische Formulierung nach Anspruch 7, dadurch gekennzeichnet, daß sie als Verdünnungsmittel zusätzlich das S—S-Isomer des R,S-Isomers der allgemeinen Formel I enthält.

9. Verwendung eines Phenethanolamins der allgemeinen Formel I gemäß einem der Ansprüche 1 bis 6 oder einer pharmazeutischen Formulierung gemäß einem der Ansprüche 7 oder 8 zur Beseitigung oder Verhinderung der Bildung von adipösem Gewebe bei Säugetieren.

10. Verfahren zur Herstellung von Phenethanolaminen der allgemeinen Formel I gemäß einem der Ansprüche 1 bis 6, dadurch gekennzeichnet, daß man.

(a) ein Styroloxid der allgemeinen Formel II

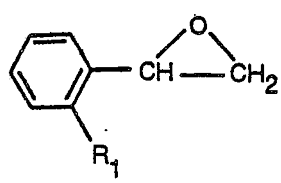

(II)

worin $R_1$ die in Anspruch 1 oder 2 angegebene Bedeutung hat, mit einem Propylaminderivat der allgemeinen Formel IV

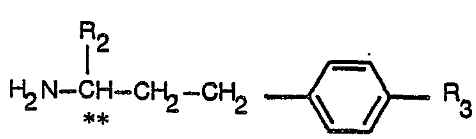

(IV)

worin $R_2$ und $R_3$ die in Anspruch 1, 3 oder 4 genannten Bedeutungen besitzen, umsetzt oder

(b) eine Verbindung der allgemeinen Formel III

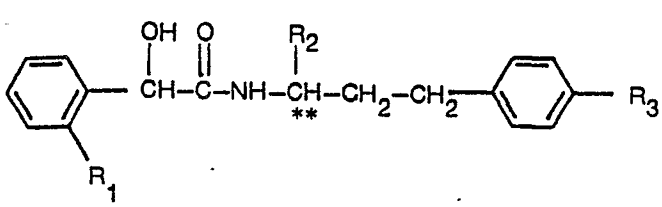

(III)

worin $R_1$ und $R_2$ die oben angegebenen Bedeutungen haben und $R_3$ ebenfalls die oben genannte Bedeutung besitzt oder zusätzlich noch für Benzyloxy steht, reduziert und die hierbei erhaltene Verbindung, falls $R_3$ Benzyloxy bedeutet, durch hydrogenolytische Abspaltung der Benzylgruppe in eine Verbindung der allgemeinen Formel I überführt, worin $R_3$ für Hydroxyl steht, und

(c) ein Produkt der allgemeinen Formel I, bei dem es sich um ein Gemisch aus einem R,S-Enantiomer und einem S,S-Enantiomer handelt, gegebenenfalls in entsprechender Weise auftrennt.

11. Tierfuttermittel, dadurch gekennzeichnet, daß es ein Phenethanolamin der allgemeinen Formel I gemäß einem der Ansprüche 1 bis 6 entweder allein oder in Verbindung mit dem S,S-Isomer enthält.